# EUROPEAN PATENT SPECIFICATION

(11) **EP 3 496 752 B1**
(45) Date of publication and mention of the grant of the patent: **18.05.2022**
(21) Application number: 17757629.5
(22) Date of filing: 11.08.2017
(51) Int. Cl.: A61K 39/395, A61K 31/00, C07K 16/22, C07K 16/28

(54) **COMBINATION THERAPY WITH A MEK INHIBITOR, A PD-1 AXIS INHIBITOR, AND A VEGF INHIBITOR**
KOMBINATIONSTHERAPIE MIT EINEM MEK-INHIBITOR, EINEM PD-1-ACHSEN-INHIBITOR UND EINEM VEGF-INHIBITOR
POLYTHÉRAPIE AVEC UN INHIBITEUR DE MEK, UN INHIBITEUR DE L'AXE PD-1 ET UN INHIBITEUR DE VEGF

(30) Priority: 12.08.2016 US 201662374437 P
(43) Date of publication of application: 19.06.2019
(73) Proprietor: Genentech, Inc., South San Francisco, CA 94080 (US)
(72) Inventor: CHOONG, Nicholas, South San Francisco, California 94080 (US)
(74) Representative: Mewburn Ellis LLP
(86) International application number: PCT/US2017/046458
(87) International publication number: WO 2018/031865

(56) References cited:
- Johanna C. Bendell et al:: "Abstract 3502: Clinical activity and safety of cobimetinib (cobi) and atezolizumab in colorectal cancer (CRC).", J. Clinical Oncology, May 2016 (2016-05), XP002774645, Retrieved from the Internet: URL:http://ascopubs.org/doi/abs/10.1200/JC O.2016.34.15_suppl.3502 [retrieved on 2017-10-02]
- BEVERLY L. FALCON ET AL: "Antagonist antibodies to vascular endothelial growth factor receptor 2 (VEGFR-2) as anti-angiogenic agents", PHARMACOLOGY AND THERAPEUTICS., 1 June 2016 (2016-06-01), XP055285941, GB ISSN: 0163-7258, DOI: 10.1016/j.pharmthera.2016.06.001
- A. SARTORE-BIANCHI ET AL: "Challenging chemoresistant metastatic colorectal cancer: therapeutic strategies from the clinic and from the laboratory", ANNALS OF ONCOLOGY., vol. 127, no. 8, 6 May 2016 (2016-05-06), pages 1456-1466, XP055415285, NL ISSN: 0923-7534, DOI: 10.1093/annonc/mdw191
- KM MORRISSEY ET AL: "Immunotherapy and Novel Combinations in Oncology: Current Landscape, Challenges, and Opportunities : Immunotherapy and Novel Combinations in Oncology", CLINICAL AND TRANSLATIONAL SCIENCE - CTS, vol. 9, no. 2, 30 March 2016 (2016-03-30), pages 89-104, XP055415286, US ISSN: 1752-8054, DOI: 10.1111/cts.12391
- Anonymous: "Study of Cobimetinib in Combination With Atezolizumab and Bevacizumab in Participants With Gastrointestinal and Other Tumors", ClinicalTrials.gov , 23 August 2016 (2016-08-23), XP002774646, Retrieved from the Internet: URL:https://clinicaltrials.gov/ct2/show/NC T02876224 [retrieved on 2017-10-02]
- MAIO ET AL: CURR PHARM DES, vol. 20, 2014, pages 3901-3913,
- BENDELL ET AL: ANNALS OF ONCOLOGY, vol. 30, October 2019 (2019-10), pages v227-v228,

## Description

### FIELD OF THE INVENTION

The field of the invention relates generally to cancer therapy with a combination of a MEK inhibitor, a PD-1 axis inhibitor, and a VEGF inhibitor.

### BACKGROUND OF THE INVENTION

Gastrointestinal tumors are common causes of cancer-related mortality worldwide. Colorectal cancer (CRC) is the third and second most commonly diagnosed cancer in males and females, respectively, and the fourth and third leading cause of cancer mortality in males and females, respectively (Torre LA, Bray F, Siegel RL, et al., Global cancer statistics, 2012, CA Cancer J Clin. 2015;65:87-108). Among patients in the United States who are diagnosed with CRC each year, about 40% are diagnosed with early stage disease, about 40% are diagnosed with regional disease, and about 20% are diagnosed with distant metastases, with five-year survival rates of 90%, 70%, and 13%, respectively, where most patients die from metastatic disease (Alberts SR and Wagman LD., Chemotherapy for colorectal cancer liver metastases, Oncologist 2008;13:1063-73; Kennecke H, Yu J, Gill S, et al., Effect of Mia and M1b Category in Metastatic Colorectal Cancer, Oncologist 2014;19:720-6; and American Cancer Society, Estimated Number∗ of New Cancer Cases and Deaths by Sex, US, 2013 [Resource on the Internet; accessed 12 November 2015].

Systemic cytotoxic chemotherapy is the mainstay of treatment for the majority of metastatic CRC (mCRC) patients and median overall survival is only around 30 months. Anti-vascular endothelial growth factor (VEGF) therapies such as bevacizumab, ramucirumab, and ziv-aflibercept, and anti-epidermal growth factor receptor monoclonal antibodies (mAb) such as cetuximab and panitumumab can be used in combination with chemotherapy in first, second, and third line therapies, though treatment combinations vary by region (Petrelli F, Coinu A, Ghilardi M, et. al. Efficacy of Oxaliplatin-based Chemotherapy + Bevacizumab as First-line Treatment for Advanced Colorectal Cancer. Am J Clin Oncol 2015; 38:227-233).

Despite recent advances, mCRC remains an incurable disease. Patients with mCRC have a continuous decrease in the disease control period with each further line of therapy, until the disease becomes refractory, and the patient succumbs to the cancer. Myelosuppression, gastrointestinal toxicity, asthenia/fatigue, peripheral neurotoxicity, and cutaneous toxicities (including hand and foot syndrome) are commonly observed adverse events in patients with mCRC who are receiving treatment. These adverse events can significantly impact on patient's quality of life.

A need therefore exists for improved therapies for CRC and metastatic CRC. The invention is defined in the appended claims.

### BRIEF DESCRIPTION OF THE INVENTION

The present disclosure provides a cancer therapy drug combination comprising: (i) a MEK inhibitor in a dose of from 20 mg to 100 mg, from 40 mg to 80 mg, or about 80 mg; (ii) a PD-1 axis inhibitor in a dose of from 400 mg to 1200 mg, from 600 mg to 1000 mg, from 700 mg to 900 mg, or 840 mg; and (iii) a VEGF inhibitor in a dose of from 5 mg/kg to 15 mg/kg, from 5 mg/kg to 10 mg/kg, 5 mg/kg, 10 mg/kg or 15 mg/kg. In the invention, the MEK inhibitor is cobimetinib or a pharmaceutically acceptable salt thereof; the PD-1 axis inhibitor is atezolizumab; and, the VEGF inhibitor is bevacizumab.

### BRIEF DESCRIPTION OF THE DRAWINGS

Figure 1 shows a study schema for the run-in and expansion cohorts of a clinical trial.
Figure 2 shows a study schema for the biopsy cohort of a clinical trial.

### DETAILED DESCRIPTION OF THE INVENTION

The present invention is directed to the treatment of cancer with the cobimetinib or a pharmaceutically acceptable salt thereof, atezolizumab and bevacizumab. The cancer is CRC, and more particularly metastatic CRC (mCRC). It is believed that the simultaneous inhibition of MEK, VEGF signaling, and the PD-1 axis, such as PD-L1, thereby targeting the tumor in a multi-factorial fashion, will enhance the efficacy of the immunotherapy component in patients with CRC. In some aspects, it is believed the combination therapy will enhance the efficacy of the immunotherapy component in patients with CRC who have received at least one prior line of therapy containing a fluoropyrimidine and oxaliplatin or irinotecan.

It is further believed that adding bevacizumab to cobimetinib and atezolizumab will offer to patients with CRC an active treatment with reduced toxicity compared with chemotherapy based regimens.

Because the mechanism of action of the combination therapy of the present invention differs from the traditional chemotherapy regimens, it is further believed that the activity of further standard therapies will not be significantly affected. This will allow patients with progressive disease to continue treatment.

### Definitions

Any references in the description to methods of treatment refer to the compound, pharmaceutical compositions and medicaments of the invention for use in a method of treatment of the human or animal body by therapy.

As used herein, "colorectal cancer" (CRC) refers to colon cancer, rectal cancer, and colorectal cancer (i.e. cancer of both the colon and rectal areas).

As used herein, the term "cancer" refers to or describes the physiological condition in mammals that is typically characterized by unregulated cell growth. A "tumor" comprises one or more cancerous cells.

As used herein, the terms "patient" and "subject" refer to animals such as mammals, including, but not limited to, primates (e.g., humans), cows, sheep, goats, horses, dogs, cats, rabbits, rats, mice and the like. In certain aspects, the patient or subject is a human.

As used herein, the term "treatment" refers to clinical intervention designed to alter the natural course of the individual or cell being treated during the course of clinical pathology. Desirable effects of treatment include decreasing the rate of disease progression, ameliorating or palliating the disease state, and remission or improved prognosis. For example, an individual is successfully "treated" if one or more symptoms associated with cancer are mitigated or eliminated, including, but are not limited to, reducing the proliferation of (or destroying) cancerous cells, decreasing symptoms resulting from the disease, increasing the quality of life of those suffering from the disease, decreasing the dose of other medications required to treat the disease, and/or prolonging survival of individuals.

As used herein, the phrase "therapeutically effective amount" refers to an amount of one or more drug compounds that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can be measured, for example, by assessing the overall response rate (ORR). A therapeutically effective amount herein may vary according to factors such as the disease state, age, sex, and weight of the patient, and the ability of the agent to elicit a desired response in the individual. A therapeutically effective amount is also one in which a toxic or detrimental effect of the treatment is outweighed by the therapeutically beneficial effect. For prophylactic use, beneficial or desired results include results such as eliminating or reducing the risk, lessening the severity, or delaying the onset of the disease, including biochemical, histological and/or behavioral symptoms of the disease, its complications and intermediate pathological phenotypes presenting during development of the disease. For therapeutic use, beneficial or desired results include clinical results such as decreasing one or more symptoms resulting from the disease, increasing the quality of life of those suffering from the disease, decreasing the dose of other medications required to treat the disease, and enhancing effect of another medication such as via targeting, delaying the progression of the disease, and/or prolonging survival. In the case of a cancer or a tumor, a therapeutically effective amount of the drug may have the effect in reducing the number of cancer cells; reducing the tumor size; inhibiting (i.e., slow to some extent or desirably stop) cancer cell infiltration into peripheral organs; inhibit (i.e., slow to some extent and desirably stop) tumor metastasis; inhibiting to some extent tumor growth; and/or relieving to some extent one or more of the symptoms associated with the disorder. A therapeutically effective amount can be administered in one or more administrations. For purposes of this invention, a therapeutically effective amount of drug, compound, or pharmaceutical composition is an amount sufficient to accomplish prophylactic or therapeutic treatment either directly or indirectly. As is understood in the clinical context, a therapeutically effective amount of a drug, compound, or pharmaceutical composition may or may not be achieved in combination with another drug, compound, or pharmaceutical composition. Thus, a therapeutically effective amount may be considered in the context of administering one or more therapeutic agents, and a single agent may be considered to be given in a therapeutically effective amount if, in combination with one or more other agents, a desirable result may be or is achieved.

As used herein, "in combination with" refers to administration of one treatment modality in addition to another treatment modality. As such, "in combination with" refers to administration of one treatment modality before, during, or after administration of the other treatment modality to the individual.

As used herein, the term "pharmaceutical formulation" refers to a preparation which is in such form as to permit the biological activity of the active ingredient to be effective, and which contains no additional components which are unacceptably toxic to a subject to which the formulation would be administered. Such formulations are sterile. "Pharmaceutically acceptable" excipients (vehicles, additives) are those which can reasonably be administered to a subject mammal to provide an effective dose of the active ingredient employed.

As used herein, "immunohistochemistry" (IHC) refers to the process of detecting antigens (e.g., proteins) in cells of a tissue section by exploiting the principle of antibodies binding specifically to antigens in biological tissues. Immunohistochemical staining may be used in the diagnosis of abnormal cells such as those found in cancerous tumors. Specific molecular markers are characteristic of particular cellular events such as proliferation or cell death (apoptosis). IHC may also be used to understand the distribution and localization of biomarkers and differentially expressed proteins in different parts of a biological tissue. Antibodies or antisera, such as polyclonal antisera and monoclonal antibodies specific for each marker, are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. In one visualization method, an antibody is conjugated to an enzyme, such as peroxidase, that can catalyze a color-producing reaction (see immunoperoxidase staining). In another visualization method, the antibody can also be tagged to a fluorophore, such as fluorescein or rhodamine (see immunofluorescence). Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

As used herein, "anti-therapeutic antibody assessment" (ATA) refers to an immunogenicity evaluation using a risk-based immunogenicity strategy as detailed in Rosenberg AS, Worobec AS., A risk-based approach to immunogenicity concerns of therapeutic protein products, BioPharm Intl 2004;17:34-42; and Koren E, Smith HW, Shores E, et al., Recommendations on risk-based strategies for detection and characterization of antibodies against biotechnology products, J Immuno Methods 2008; 333:1-9) to characterize ATA responses.

As used herein, Cₘₐₓ refers to maximum plasma concentration.

As used herein, Cₘᵢₙ refers to minimum plasma concentration.

As used herein "area under concentration curve" (AUC) refers to the area under a fitted plasma concentration versus time curve. AUC_{0-∞} refers to area under curve baseline - infinity. AUC_{0-T} is total exposure.

As used herein "Response Evaluation Criteria in Solid Tumors" (RECIST) v1.1 refers to tumor response criteria conventions as detailed by Eisenhauer, EA, et al., New response evaluation criteria in solid tumours: Revised RECIST guideline (version 1.1), Eur J Cancer 2009:45:228-247; by Topalian SL, et al., Safety, activity, and immune correlates of anti-PD-L1 antibody in cancer, N Engl J Med 2012:366:2443-54; and by Wolchok JD, et al., Guidelines for the evaluation of immunetherapy activity in solid tumors: immune-related response criteria, Clin Can Res 2009;15:7412-20.

As used herein "Immune-Modified RECIST" (irRC) refers to criteria derived from RECIST v1.1 conventions (Eisenhauer, EA, et al., (2009)) and immune response criteria as detailed by Nishino M, et al., Optimizing immune-related tumor response assessment: does reducing the number of lesions impact response assessment in melanoma patients treated with ipilimumab, J Immunother Can 2014;2:17; and Nishino M, Giobbie-Hurder A, Gargano M et al., Developing a common language for tumor response to immunotherapy: immune-related response criteria using unidimensional measurements, Clin Can Res 2013;19:3936-43. .). Unless otherwise specified, RECIST v1.1 conventions apply.

As used herein "inhibit" refers to a decrease the activity of the target enzyme, as compared to the activity of that enzyme in the absence of the inhibitor. In some aspects, the term "inhibit" means a decrease in activity of at least about 5%, at least about 10%, at least about 20%, at least about 25%, at least about 50%, at least about 60%, at least about 70%, at least about 80%, at least about 90%, or at least about 95%. In other aspects, inhibit means a decrease in activity of about 5% to about 25%, about 25% to about 50%, about 50% to about 75%, or about 75% to 100%. In some aspects, inhibit means a decrease in activity of about 95% to 100%, e.g., a decrease in activity of 95%, 96%, 97%, 98%, 99%, or 100%. Such decreases can be measured using a variety of techniques that would be recognizable by one of skill in the art.

As used herein, "progression free survival" (PFS) refers to the time from the treatment of the disease to the first occurrence of disease progression or relapse as determined by the investigator using RECIST v1.1.

As used herein, "overall survival" (OS) refers to the time from randomization to death from any cause.

As used herein, "partial response" (PR) refers to at least a 30% decrease in the sum of diameters of target lesions, taking as reference the baseline sum of diameters.

As used herein, "delaying the progression" of a disease means to defer, hinder, slow, retard, stabilize, and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

As used herein "sustained response" refers to the sustained effect on reducing tumor growth after cessation of a treatment. For example, the tumor size may remain to be the same or smaller as compared to the size at the beginning of the administration phase. In some aspects, the sustained response has a duration at least the same as the treatment duration, at least 1.5x, 2x, 2.5x, or 3x of the length of the treatment duration.

As used herein, "reducing or inhibiting cancer relapse" means to reduce or inhibit tumor or cancer relapse or tumor or cancer progression. As disclosed herein, cancer relapse and/or cancer progression include, without limitation, cancer metastasis.

As used herein, "complete response" (CR) refers to the disappearance of all target lesions. Any pathological lymph nodes (whether target or non-target) have a reducing in short axis to less than 10 mm

As used herein, "progressive disease" (PD) refers to at least a 20% increase in the sum of diameters of target lesions, taking as reference the smallest sum on study (nadir), including baseline and an absolute increase of at least 5 mm. The appearance of one or more new lesions is also considered progression.

As used herein, "stable disease" (SD) refers to neither sufficient shrinkage to qualify for PR nor sufficient increase to qualify for PD, taking as reference the smallest sum on study.

As used herein, "overall response rate" (ORR) refers to the rate of a PR or CR occurring after randomization and confirmed ≥ 28 days later as determined by the investigator using RECIST v1.1.

As used herein, "unconfirmed overall response rate" (ORR_uc) refers to the rate of a PR or CR occurring after randomization as determined by the investigator using RECIST v1.1 where confirmation is not required.

As used herein, "duration of response" (DOR) refers to the time from the first occurrence of a documented objective response to the time of relapse, as determined by the investigator using RECIST v1.1 or death from any cause during the study, whichever occurs first.

As used herein, "National Cancer Institute Common Terminology Criteria for Adverse Events" (NCI CTCAE) refers to Common Terminology Criteria for Adverse Effect, Version 4.0, published May 28, 2009 (v4.03: June 14, 2010) by the U.S. Department of Health and Human Services, National Institutes of Health, National Cancer Institute

As used herein, "Functional Assessment of Cancer Therapy General" (FACT-G) refers to a validated and reliable 27-item questionnaire comprised of four subscales that measure physical (7 items), social/family (7 items), emotional (6 items) and functional wellbeing (7 items), and is considered appropriate for use with patients with any form of cancer (Cella DF, Tulsky DS, Gray G, Sarafian B, Linn E, Bonomi AE et al., The Functional Assessment of Cancer Therapy scale: development and validation of the general measure, Journal of Clinical Oncology 1993; 11(3 Suppl.2):570-9; and Webster, K., Odom, L., Peterman, A., Lent, L., Cella, D., The Functional Assessment of Chronic Illness Therapy (FACIT) measurement system: Validation of version 4 of the core questionnaire, Quality of Life Research 1999, 8(7):604.). Patients assess how true each statement has been for them in the previous 7 days on a five-point scale (0, not at all; 1, a little bit; 2, somewhat; 3, quite a bit; 4, very much).

As used herein, the term "MEK inhibitor(s)" refers to a molecule that inhibits a MEK, such as the mitogen-activated protein kinase enzymes MEK1 (also known as MAP2K1), or MEK2 (also known as MAP2K2). A MEK inhibitor may be used to affect the MAPK/ERK pathway that may be over active in some cancers, such as CRC. MEK inhibitors have been extensively reviewed (S. Price, Putative Allosteric and MEK 2 inhibitors, Expert Opin. Ther. Patents, 2008 18(6):603; J. I. Trujillo, MEK Inhibitors: a patent review 2008-2010,Expert Opin. Ther. Patents 2011 21(7):1045).

As used herein, the term "PD-1 axis inhibitor" or "binding antagonist" refers to a molecule that inhibits the interaction of a PD-1 axis binding partner with either one or more of its binding partner, so as to remove T-cell dysfunction resulting from signaling on the PD-1 signaling axis - with a result being to restore or enhance T-cell function (e.g., proliferation, cytokine production, target cell killing). As used herein, a PD-1 axis inhibitor includes a PD-1 inhibitor, a PD-L1 inhibitor, and a PD-L2 inhibitor.

As used herein, the term "PD-1 inhibitor" or "binding antagonist" refers to a molecule that decreases, blocks, inhibits, abrogates or interferes with signal transduction resulting from the interaction of PD-1 with one or more of its binding partners, such as PD-L1 cases and PD-L2. In some cases, the PD-1 inhibitor is a molecule that inhibits the binding of PD-1 to one or more of its binding partners. In a specific aspect, the PD-1 inhibitor inhibits the binding of PD-1 to PD-L1 and/or PD-L2. For example, PD-1 inhibitors include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In one case, a PD-1 inhibitor reduces the negative co-stimulatory signal mediated by or through cell surface proteins expressed on T lymphocytes mediated signaling through PD-1 so as render a dysfunctional T-cell less dysfunctional (e.g., enhancing effector responses to antigen cases recognition). In some cases, the PD-1 inhibitor is an anti-PD-1 antibody.

As used herein, the term "PD-L1 inhibitor" or "binding antagonist" refers to a molecule that decreases, blocks, inhibits, abrogates or interferes with signal transduction resulting from the interaction of PD-L1 with either one or more of its binding partners, such as PD-1, B7-1. In some cases, a PD-L1 inhibitor is a molecule that inhibits the binding of PD-L1 to its binding partners. In a specific aspect, the PD-L1 inhibitor inhibits binding of PD-L1 to PD-1 and/or B7-1. In some cases, the PD-L1 inhibitor include anti-PD-Ll antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, case B7-1. In one case, a PD-L1 inhibitor reduces the negative co-stimulatory signal mediated by or through cell surface proteins expressed on T lymphocytes mediated signaling through PD-L1 so as to render a dysfunctional T-cell less dysfunctional (e.g., enhancing cases effector responses to antigen recognition). In some cases, a PD-L1 inhibitor is an anti-PD-L1 antibody.

As used herein, the term "PD-L2 inhibitor" or "binding antagonist" refers to a molecule that decreases, blocks, inhibits, abrogates or interferes with signal transduction resulting from the interaction of PD-L2 with either one or more of its binding partners, such as cases PD-1. In some cases, a PD-L2 inhibitor is a molecule that inhibits the binding of PD-L2 to one or more of its binding partners. In a specific aspect, the PD-L2 inhibitor inhibits cases binding of PD-L2 to PD-1. In some cases, the PD-L2 inhibitor include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In one case, a PD-L2 inhibitor reduces the negative co-stimulatory signal mediated by or through cell surface proteins expressed on T lymphocytes mediated signaling through PD-L2 so as render a dysfunctional T-cell less dysfunctional (e.g., enhancing effector cases responses to antigen recognition). In some cases, a PD-L2 inhibitor is an immunoadhesin.

As used herein, the term "dysfunction" in the context of immune dysfunction, refers to a state of reduced immune responsiveness to antigenic stimulation. The term includes the common elements of both exhaustion and/or anergy in which antigen recognition may occur, but the ensuing immune response is ineffective to control infection or tumor growth. As used herein, the term "dysfunctional" also includes refractory or unresponsive to antigen recognition, specifically, impaired capacity to translate antigen recognition into down-stream T-cell effector functions, such as proliferation, cytokine production (e.g., IL-2) and/or target cell killing.

As used herein, the term "anergy" refers to the state of unresponsiveness to antigen stimulation resulting from incomplete or insufficient signals delivered through the T-cell receptor (e.g. increase in intracellular Ca+2 in the absence of ras-activation). T cell anergy can also result upon stimulation with antigen in the absence of co-stimulation, resulting in the cell becoming refractory to subsequent activation by the antigen even in the context of co-stimulation. The unresponsive state can often be overriden by the presence of Interleukin-2. Anergic T-cells do not undergo clonal expansion and/or acquire effector functions.

As used herein, the term "exhaustion" refers to T cell exhaustion as a state of T cell dysfunction that arises from sustained TCR signaling that occurs during many chronic infections and cancer. It is distinguished from anergy in that it arises not through incomplete or deficient signaling, but from sustained signaling. It is defined by poor effector function, sustained expression of inhibitory receptors and a transcriptional state distinct from that of functional effector or memory T cells. Exhaustion prevents optimal control of infection and tumors. Exhaustion can result from both extrinsic negative regulatory pathways (e.g., immunoregulatory cytokines) as well as cell intrinsic negative regulatory (costimulatory) pathways (PD-1, B7-H3, B7-H4, etc.).

"Enhancing T-cell function" means to induce, cause or stimulate a T-cell to have a sustained or amplified biological function, or renew or reactivate exhausted or inactive T-cells. Examples of enhancing T-cell function include: increased secretion of gamma-interferon from CD8+ T-cells, increased proliferation, increased antigen responsiveness (e.g., viral, pathogen, or tumor clearance) relative to such levels before the intervention. In one embodiment, the level of enhancement is as least 50%, alternatively 60%, 70%, 80%, 90%, 100%, 120%, 150%, 200%. The manner of measuring this enhancement is known to one of ordinary skill in the art.

A "T cell dysfunctional disorder" is a disorder or condition of T-cells characterized by decreased responsiveness to antigenic stimulation. In a particular embodiment, a T-cell dysfunctional disorder is a disorder that is specifically associated with inappropriate increased signaling through PD-1. In another embodiment, a T-cell dysfunctional disorder is one in which T-cells are anergic or have decreased ability to secrete cytokines, proliferate, or execute cytolytic activity. In a specific aspect, the decreased responsiveness results in ineffective control of a pathogen or tumor expressing an immunogen. Examples of T cell dysfunctional disorders characterized by T-cell dysfunction include unresolved acute infection, chronic infection and tumor immunity.

As used herein, "VEGF" refers to the 165-amino acid human vascular endothelial cell growth factor and related 121-, 189-, and 206-amino acid human vascular endothelial cell growth factors, as described by Leung et al. (1989) Science 246:1306, and Houck et al. (1991) Mol. Endocrin, 5:1806, together with the naturally occurring allelic and processed forms thereof. The term "VEGF" also refers to VEGFs from non-human species such as mouse, rat, or primate. Sometimes the VEGF from a specific species are indicated by terms such as hVEGF for human VEGF, mVEGF for murine VEGF, etc. The term "VEGF" is also used to refer to truncated forms of the polypeptide comprising amino acids 8 to 109 or 1 to 109 of the 165-amino acid human vascular endothelial cell growth factor. Reference to any such forms of VEGF may be identified in the present application, e.g., by "VEGF (8-109)," "VEGF (1-109)" or "VEGF.sub.165." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

As used herein, a "VEGF inhibitor" refers to a molecule capable of neutralizing, blocking, inhibiting, abrogating, reducing or interfering with VEGF activities including its binding to one or more VEGF receptors. VEGF antagonists include anti-VEGF antibodies and antigen-binding fragments thereof, receptor molecules and derivatives which bind specifically to VEGF thereby sequestering its binding to one or more receptors, anti-VEGF receptor antibodies and VEGF receptor antagonists such as small molecule inhibitors of the VEGFR tyrosine kinases, and fusions proteins, e.g., VEGF-Trap (Regeneron), VEGF.sub.121-gelonin (Peregrine). VEGF antagonists also include antagonist variants of VEGF, antisense molecules directed to VEGF, RNA aptamers specific to VEGF, and ribozymes against VEGF or VEGF receptors. Antagonists of VEGF act by interfering with the binding of VEGF to a cellular receptor, by incapacitating or killing cells which have been activated by VEGF, or by interfering with vascular endothelial cell activation after VEGF binding to a cellular receptor. All such points of intervention by a VEGF antagonist shall be considered equivalent for purposes of this invention. Preferred VEGF antagonists are anti-VEGF antagonistic antibodies capable of inhibiting one or more of the biological activities of VEGF, for example, its mitogenic, angiogenic or vascular permeability activity. Anti-VEGF antagonistic antibodies include, antibodies A4.6.1, rhuMab VEGF (bevacizumab), Y0317 (ranibizumab), G6, B20, 2C3, and others as described in, for example, WO98/45331, US2003/0190317, U.S. Pat. Nos. 6,582,959 and 6,703,020; WO98/45332; WO 96/30046; WO94/10202; WO2005/044853; EP 0666868B1; and Popkov et al., Journal of Immunological Methods 288:149-164 (2004).

As used herein, the term "package insert" refers to instructions customarily included in commercial packages of therapeutic products, that contain information about the indications, usage, dosage, administration, contraindications and/or warnings concerning the use of such therapeutic products.

The term "pharmaceutically acceptable salts" denotes salts which are not biologically or otherwise undesirable. Pharmaceutically acceptable salts include both acid and base addition salts. The phrase "pharmaceutically acceptable" indicates that the substance or composition is compatible chemically and/or toxicologically, with the other ingredients comprising a formulation, and/or the mammal being treated therewith. Acid addition salts are formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, malonic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid "mesylate", ethanesulfonic acid, p-toluenesulfonic acid, and salicyclic acid. Base addition salts are formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, and polyamine resins.

The term "antibody" herein is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

An "isolated" antibody is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. In some embodiments, an antibody is purified (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody includes the antibody in situ within recombinant cells since at least one component of the antibody's natural environment will not be present. Ordinarily, however, isolated antibody will be prepared by at least one purification step.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

The term "constant domain" refers to the portion of an immunoglobulin molecule having a more conserved amino acid sequence relative to the other portion of the immunoglobulin, the variable domain, which contains the antigen binding site. The constant domain contains the CH1, CH2 and CH3 domains (collectively, CH) of the heavy chain and the CHL (or CL) domain of the light chain.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions (HVRs) both in the light-chain and the heavy-chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen-binding site of antibodies (see Kabat et al., Sequences of Proteins of Immunological Interest, Fifth Edition, National Institute of Health, Bethesda, Md. (1991)). The constant domains are not involved directly in the binding of an antibody to an antigen, but exhibit various effector functions, such as participation of the antibody in antibody-dependent cellular toxicity.

The "light chains" of antibodies (immunoglobulins) from any mammalian species can be assigned to one of two clearly distinct types, called kappa ("κ") and lambda ("λ"), based on the amino acid sequences of their constant domains.

The term IgG "isotype" or "subclass" as used herein is meant any of the subclasses of immunoglobulins defined by the chemical and antigenic characteristics of their constant regions.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, γ, ε, γ, and µ, respectively. The subunit structures and three-dimensional configurations of different classes of immunoglobulins are well known and described generally in, for example, Abbas et al. Cellular and Mol. Immunology, 4th ed. (W.B. Saunders, Co., 2000). An antibody may be part of a larger fusion molecule, formed by covalent or non-covalent association of the antibody with one or more other proteins or peptides.

The terms "full length antibody," "intact antibody" and "whole antibody" are used herein interchangeably to refer to an antibody in its substantially intact form, not antibody fragments as defined below. The terms particularly refer to an antibody with heavy chains that contain an Fc region.

A "naked antibody" for the purposes herein is an antibody that is not conjugated to a cytotoxic moiety or radiolabel.

"Antibody fragments" comprise a portion of an intact antibody, preferably comprising the antigen binding region thereof. In some embodiments, the antibody fragment described herein is an antigen-binding fragment. Examples of antibody fragments include Fab, Fab', F(ab')2, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv, see, e.g., Pluckthün, in The Pharmacology of Monoclonal Antibodies, vol. 113, Rosenburg and Moore eds., (Springer-Verlag, New York, 1994), pp. 269-315.

The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

The term "monoclonal antibody" as used herein refers to an antibody obtained from a population of substantially homogeneous antibodies, e.g., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity in vivo, to create a multispecific antibody, etc., and that an antibody comprising the altered target binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the invention may be made by a variety of techniques, including, for example, the hybridoma method (e.g., Kohler and Milstein, Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Pat. No. 4,816,567), phage-display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004), and technologies for producing human or human-like antibodies in animals that have parts or all of the human immunoglobulin loci or genes encoding human immunoglobulin sequences (see, e.g., WO 1998/24893; WO 1996/34096; WO 1996/33735; WO 1991/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Pat. Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see, e.g., U.S. Pat. No. 4,816,567; and Morrison et al., Proc. Natl. Acad. Sci. USA 81:6851-6855 (1984)). Chimeric antibodies include PRIMATTZED^{®} antibodies wherein the antigen-binding region of the antibody is derived from an antibody produced by, e.g., immunizing macaque monkeys with the antigen of interest.

"Humanized" forms of non-human (e.g., murine) antibodies are chimeric antibodies that contain minimal sequence derived from non-human immunoglobulin. In one embodiment, a humanized antibody is a human immunoglobulin (recipient antibody) in which residues from a HVR of the recipient are replaced by residues from a HVR of a non-human species (donor antibody) such as mouse, rat, rabbit, or nonhuman primate having the desired specificity, affinity, and/or capacity. In some instances, FR residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see, e.g., Jones et al., Nature 321:522-525 (1986); Riechmann et al., Nature 332:323-329 (1988); and Presta, Curr. Op. Struct. Biol. 2:593-596 (1992). See also, e.g., Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSETM technology). See also, for example, Li et al., Proc. Natl. Acad. Sci. USA, 103:3557-3562 (2006) regarding human antibodies generated via a human B-cell hybridoma technology.

A "species-dependent antibody" is one which has a stronger binding affinity for an antigen from a first mammalian species than it has for a homologue of that antigen from a second mammalian species. Normally, the species-dependent antibody "binds specifically" to a human antigen (e.g., has a binding affinity (Kd) value of no more than about 1×10⁻⁷ M, preferably no more than about 1×10⁻⁸ M and preferably no more than about 1×10⁻⁹ M) but has a binding affinity for a homologue of the antigen from a second nonhuman mammalian species which is at least about 50 fold, or at least about 500 fold, or at least about 1000 fold, weaker than its binding affinity for the human antigen. The species-dependent antibody can be any of the various types of antibodies as defined above, but preferably is a humanized or human antibody.

The term "hypervariable region," "HVR," or "HV," when used herein refers to the regions of an antibody variable domain which are hypervariable in sequence and/or form structurally defined loops. Generally, antibodies comprise six HVRs; three in the VH (HI, H2, H3), and three in the VL (L1, L2, L3). In native antibodies, H3 and L3 display the most diversity of the six HVRs, and H3 in particular is believed to play a unique role in conferring fine specificity to antibodies. See, e.g., Xu et al., Immunity 13:37-45 (2000); Johnson and Wu, in Methods in Molecular Biology 248:1-25 (Lo, ed., Human Press, Totowa, N.J., 2003). Indeed, naturally occurring camelid antibodies consisting of a heavy chain only are functional and stable in the absence of light chain. See, e.g., Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat Numbering) |
| H1 | H31-H35 | H26-H35 | H26-H32 | H30-H35 (Chothia Numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (LI), 46-56 or 50-56 (L2) and 89-97 or 89-96 (L3) in the VL and 26-35 (HI), 50-65 or 49-65 (H2) and 93-102, 94-102, or 95-102 (H3) in the VH. The variable domain residues are numbered according to Kabat et al., supra, for each of these definitions.

"Framework" or "FR" residues are those variable domain residues other than the HVR residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., supra. Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (e.g., Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., supra). The "EU index as in Kabat" refers to the residue numbering of the human IgG1 EU antibody.

The expression "linear antibodies" refers to the antibodies described in Zapata et al. (1995 Protein Eng, 8(10): 1057-1062). Briefly, these antibodies comprise a pair of tandem Fd segments (VH-CH1-VH-CH1) which, together with complementary light chain polypeptides, form a pair of antigen binding regions. Linear antibodies can be bispecific or monospecific.

As use herein, the term "binds", "specifically binds to" or is "specific for" refers to measurable and reproducible interactions such as binding between a target and an antibody, which is determinative of the presence of the target in the presence of a heterogeneous population of molecules including biological molecules. For example, an antibody that binds to or specifically binds to a target (which can be an epitope) is an antibody that binds this target with greater affinity, avidity, more readily, and/or with greater duration than it binds to other targets. In one embodiment, the extent of binding of an antibody to an unrelated target is less than about 10% of the binding of the antibody to the target as measured, e.g., by a radioimmunoassay (RIA). In certain embodiments, an antibody that specifically binds to a target has a dissociation constant (Kd) of ≤ 1µM, ≤ 100 nM, ≤ 10 nM, ≤ 1 nM, or ≤ 0.1 nM. In certain embodiments, an antibody specifically binds to an epitope on a protein that is conserved among the protein from different species. In another embodiment, specific binding can include, but does not require exclusive binding.

The term "detection" includes any means of detecting, including direct and indirect detection.

The term "biomarker" as used herein refers to an indicator, e.g., predictive, diagnostic, and/or prognostic, which can be detected in a sample. The biomarker may serve as an indicator of a particular subtype of a disease or disorder (e.g., cancer) characterized by certain, molecular, pathological, histological, and/or clinical features. In some embodiments, a biomarker is a gene. Biomarkers include, but are not limited to, polynucleotides (e.g., DNA, and/or RNA), polynucleotide copy number alterations (e.g., DNA copy numbers), polypeptides, polypeptide and polynucleotide modifications (e.g. posttranslational modifications), carbohydrates, and/or glycolipid-based molecular markers.

### Therapeutic Agents

The present disclosure uses the combination of , the MEK inhibitor cobimetinib or a pharmaceutically acceptable salt thereof; the PD-1 axis inhibitor which is the PD-L1 inhibitor atezolizumab; and, the VEGF inhibitor bevacizumab. In some other aspects, cobimetinib is Cotellic^{®}, atezolizumab is Tecentriq^{®}, and/or bevacizumab is Avastin^{®}.

The presently disclosed compounds may be administered in any suitable manner known in the art. In some aspects, the compounds may be administered intravenously, intramuscularly, subcutaneously, topically, orally, transdermally, intraperitoneally, intraorbitally, by implantation, by inhalation, intrathecally, intraventricularly, intratumorally, or intranasally.

It is understood that appropriate doses of the active compound depends upon a number of factors within the knowledge of the ordinarily skilled physician. The dose(s) of the active compound will vary, for example, depending upon the age, body weight, general health, gender, and diet of the subject, the time of administration, the route of administration, the rate of excretion, and any drug combination.

It will also be appreciated that the effective dosage of the compound of the present disclosure, or a pharmaceutically acceptable salts, prodrugs, metabolites, or derivatives thereof used for treatment may increase or decrease over the course of a particular treatment. Changes in dosage may result and become apparent from the results of diagnostic assays.

### MEK Inhibitor

In the invention, the MEK inhibitor is cobimetinib or a pharmaceutically acceptable salt thereof (e.g., Cotellic^{®}) having the chemical name (S)[3,4-Difluoro-2-(2-fluoro-4-iodophenylamino)phenyl][3-hydroxy-3-(piperidin-2-yl]azetidin-1-yl) methanone, and having the below structure: 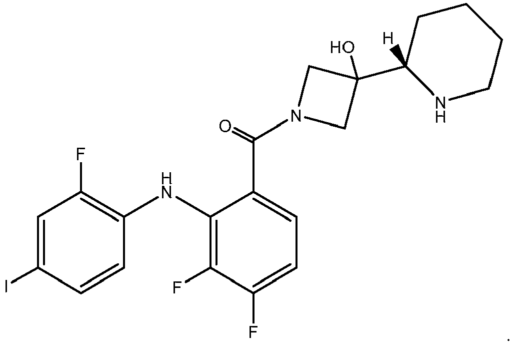 Cotellic^{®} is the fumarate salt of cobimetinib. Cobimetinib is described in U.S. Patent Nos. 7,803,839 and 8,362,002. Cobimetinib is a reversible, potent, and highly selective inhibitor of MEK1 and MEK2 (central components of the RAS/RAF/MEK/ERK (MAPK)) pathway and has single agent anti-tumor activity in multiple human cancer models.

Cobimetinib inhibits proliferation of a variety of human tumor cell lines through inhibition of MEK1 and MEK2. In addition, cobimetinib inhibits ERK phosphorylation in xenograft tumor models and stimulates apoptosis. Cobimetinib accumulates in tumor xenografts and remains at high concentrations in the tumor after plasma concentrations have declined. The activity of cobimetinib to inhibit ERK1 phosphorylation is more closely correlated with its concentration in tumor tissue than in plasma; in general, there is a good correlation between reduced ERK1 phosphorylation and efficacy in tumor xenograft models. Tumor regression has been observed in several human tumor xenograft models. This regression was dose dependent with up to 100% regression at the highest doses tested. The models studied include CRC, malignant melanoma, breast carcinoma, and lung carcinoma.

The pharmacokinetics (PK) of cobimetinib administered as a single agent have been characterized in cancer patients following oral administration after single and multiple dosing in the Phase Ia dose-escalation Study MEK4592g which included evaluation of a cobimetinib dose of 60 mg per day in patients who harbored a BRAF, NRAS, or KRAS mutation. Overall 6 patients (all of whom had melanoma; 6.2%) had a confirmed partial response (PR), 28 patients (28.9%) had stable disease (SD), and 40 patients (41.2%) had progressive disease. Out of the 14 colorectal cancer (CRC) patients, all patients experienced progressive disease (PD). In Stage III of Study MEK4592g, 18 patients were accrued, and best overall response was assessed for 14 of 18 patients. Four patients (22.2%) had SD as their best overall response, and 2 patients (11.1%) had unconfirmed tumor responses.

Cobimetinib has a moderate rate of absorption (median time to maximum concentration [tₘₐₓ] of 1 to 3 hours) and a mean terminal half-life (t_{1/2}) of 48.8 hours (a range of 23.1 to 80 hours). Cobimetinib binds to plasma proteins (95%) in a concentration-independent manner. Cobimetinib exhibits linear pharmacokinetics in the dose range of 0.05 mg/kg (approximately 3.5 mg/kg for 70 kg adult) to 80 mg and the absolute bioavailability was determined to be 45.9% (90% CI: 39.74%, 53.06%) in study MEK4952g in healthy subjects. Cobimetinib pharmacokinetics are not altered when administered in the fed state compared with administration in the fasted state in healthy subjects. Since food does not alter cobimetinib pharmacokinetics, cobimetinib can be administered with or without food. The proton pump inhibitor rabeprazole appears to have a minimal effect on cobimetinib pharmacokinetics, whether administered in the presence or absence of a high-fat meal compared with cobimetinib administration alone in the fasted state. Thus, increase in gastric pH does not affect cobimetinib pharmacokinetics, indicating it is not sensitive to alterations in gastric pH.

Cobimetinib salts, crystalline forms and prodrugs are within the scope of the present disclosure. Cobimetinib, preparative methods, and therapeutic uses are disclosed in International Publication Numbers WO 2007/044515, WO 2007/044615, WO 2014/027056 and WO 2014/059422. For instance, in some aspects of the present disclosure, the MEK inhibitor is crystalline hemifumarate cobimetinib polymorph Form A.

MEK inhibitor (cobimetinib) doses within the scope of the present disclosure are from 20 mg to 100 mg, from 40 mg to 80 mg, or 60 mg of the MEK inhibitor per day. In particular embodiments, the MEK inhibitor is cobimetinib, and is dosed at 60 mg, 40 mg or 20 mg.

The MEK inhibitor is suitably administered once daily. In some aspects, the MEK inhibitor is administered once daily for 21 consecutive days of a 28-day treatment cycle. In some aspects, the MEK inhibitor is administered once daily on days 1 to 21 of a 28-day treatment cycle. In some aspects, the MEK inhibitor is administered once daily on days 3 to 23 of a 28-day treatment cycle.

### PD-1 Axis Inhibitors

In accordance with the present disclosure, a PD-1 axis inhibitor may more particularly refer to a PD-1 inhibitor, a PD-L1 inhibitor, or a PD-L2 inhibitor. Alternative names for "PD-1" include CD279 and SLEB2. Alternative names for "PD-L1" include B7-H1, B7-4, CD274, and B7-H. Alternative names for "PD-L2" include B7-DC, Btdc, and CD273. In some cases, PD-1, PD-L1, and PD-L2 are human PD-1, PD-L1 and PD-L2.

In some cases the PD-1 inhibitor is a molecule that inhibits the binding of PD-1 to its ligand binding partners. In a specific aspect the PD-1 ligand binding partners are PD-L1 and/or PD-L2. In another case, a PD-L1 inhibitor is a molecule that inhibits the binding of PD-L1 to its binding partners. In a specific aspect, PD-L1 binding partners are PD-1 and/or B7-1. In another case, the PD-L2 inhibitor is a molecule that inhibits the binding of PD-L2 to its binding partners. In a specific aspect, a PD-L2 binding partner is PD-1. The inhibitor may be an antibody, an antigen binding fragment thereof, an immunoadhesin, a fusion protein, or oligopeptide.

In the invention, the PD-L1 inhibitor is the anti-PD-L1 antibody

Examples of anti-PD-Ll antibodies, and methods for making thereof are described in PCT patent application WO 2010/077634 A1 and US Patent No. 8,217,149

In the invention the PD-1 axis inhibitor is an anti-PD-Ll antibody. In some cases the invention the anti-PD-Ll antibody is capable of inhibiting binding between PD-L1 and PD-1 and/or between PD-L1 and B7-1. In some cases, the invention, the anti-PD-Ll antibody is a monoclonal antibody. In some cases, the anti-PD-Ll antibody is an antibody fragment selected from the group consisting of Fab, Fab'-SH, Fv, scFv, and (Fab')₂ fragments. In some cases, the anti-PD-Ll antibody is a humanized antibody. In some cases cases, the anti-PD-L1 antibody is a human antibody.

In the invention, the anti-PD-Ll antibody is atezolizumab, or MPDL3280A (CAS Registry Number: 1422185-06-5). In a still further embodiment, provided is an isolated anti-PD-Ll antibody comprising a heavy chain variable region comprising the heavy chain variable region amino acid sequence from EVQLVESGGGLVQPGGSLRLSCAASGFTFSDSWIHWVRQAPGKGLEWVAWISPYGGS TYYADSVKGRFTISADTSKNTAYLQMNSLRAEDTAVYYCARRHWPGGFDYWGQGTL VTVSS (SEQ ID NO:7) or EVQLVESGGGLVQPGGSLRLSCAASGFTFSDSWIHWVRQAPGKGLEWVAWI SPYGGSTYYADSVKGRFTISADTSKNTAYLQMNSLRAEDTAVYYCARRHWPGGFDY WGQGTLVTVSSASTK (SEQ ID NO:8) and a light chain variable region comprising the amino acid sequence of DIQMTQSPSSLSASVGDRVTITCRASQDVSTAVAWYQQKPGKAPKLLIY SASF LYSGVPSRFSGSGSGTDFTLTISSLQPEDFATYYCQQYLYHPATFGQGTKVEIKR (SEQ ID NO:9). In a still further embodiment, provided is an isolated anti-PD-Ll antibody comprising a heavy chain and/or a light chain sequence, wherein:
(a) the heavy chain sequence has at least 85%, at least 90%, at least 91%, at least 92%, at least 93%, at least 94%, at least 95%, at least 96%, at least 97%, at least 98%, at least 99% or 100% sequence identity to the heavy chain sequence: and/or
(b) the light chain sequences has at least 85%, at least 90%, at least 91%, at least 92%, at least 93%, at least 94%, at least 95%, at least 96%, at least 97%, at least 98%, at least 99% or 100% sequence identity to the light chain sequence:

In a still further embodiment, provided is an isolated nucleic acid encoding a light chain or a heavy chain variable region sequence of an anti-PD-Ll antibody, wherein:
(a) the heavy chain further comprises and HVR-H1, HVR-H2 and an HVR-H3 sequence having at least 85% sequence identity to GFTFSDSWIH (SEQ ID NO:24), AWISPYGGSTYYADSVKG (SEQ ID NO:25) and RHWPGGFDY (SEQ ID NO: 12), respectively, and
(b) the light chain further comprises an HVR-L1, HVR-L2 and an HVR-L3 sequence having at least 85% sequence identity to RASQDVSTAVA (SEQ ID NO:26), SASFLYS (SEQ ID NO:27) and QQYLYHPAT (SEQ ID NO:28), respectively.

In a specific aspect, the sequence identity is 86%, 87%, 88%, 89%, 90%, 91%, 92%, 93%, 94%, 95%, 96%, 97%, 98%, 99% or 100%. In one aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In yet another aspect, the framework sequences are derived from human consensus framework sequences. In a further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences is the following:

| | | |
|---|---|---|
| HC-FR1 | EVQLVESGGGLVQPGGSLRLSCAAS | (SEQ ID NO:13) |
| HC-FR2 | WVRQAPGKGLEWV | (SEQ ID NO:14) |
| HC-FR3 | RFTISADTSKNTAYLQMNSLRAEDTAVYYCAR | (SEQ ID NO:15) |
| HC-FR4 | WGQGTLVTVSA | (SEQ ID NO:16). |

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences is the following:

| | | |
|---|---|---|
| LC-FR1 | DIQMTQSPSSLSASVGDRVTITC | (SEQ ID NO:20) |
| LC-FR2 | WYQQKPGKAPKLLIY | (SEQ ID NO:21) |
| LC-FR3 | GVPSRFSGSGSGTDFTLTISSLQPEDFATYYC | (SEQ ID NO:22) |
| LC-FR4 | FGQGTKVEIKR | (SEQ ID NO:23). |

In a still further specific aspect, the antibody described herein (such as an anti-PD-1 antibody, an anti-PD-Ll antibody, or an anti-PD-L2 antibody) further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect, the minimal effector function results from production in prokaryotic cells. In a still further specific aspect the minimal effector function results from an "effector-less Fc mutation" or aglycosylation. In still a further aspect, the effector-less Fc mutation is an N297A or D265A/N297A substitution in the constant region.

In a still further aspect, provided herein are nucleic acids encoding any of the antibodies described herein. In some embodiments, the nucleic acid further comprises a vector suitable for expression of the nucleic acid encoding any of the previously described anti-PD-L1, anti-PD-1, or anti-PD-L2 antibodies. In a still further specific aspect, the vector further comprises a host cell suitable for expression of the nucleic acid. In a still further specific aspect, the host cell is a eukaryotic cell or a prokaryotic cell. In a still further specific aspect, the eukaryotic cell is a mammalian cell, such as Chinese Hamster Ovary (CHO).

The antibody or antigen binding fragment thereof, may be made using methods known in the art, for example, by a process comprising culturing a host cell containing nucleic acid encoding any of the previously described anti-PD-Ll, anti-PD-1, or anti-PD-L2 antibodies or antigen-binding fragment in a form suitable for expression, under conditions suitable to produce such antibody or fragment, and recovering the antibody or fragment.

In some embodiments, the isolated anti-PD-Ll antibody is aglycosylated. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Removal of glycosylation sites form an antibody is conveniently accomplished by altering the amino acid sequence such that one of the above-described tripeptide sequences (for N-linked glycosylation sites) is removed. The alteration may be made by substitution of an asparagine, serine or threonine residue within the glycosylation site another amino acid residue (e.g., glycine, alanine or a conservative substitution).

In this regard it is to be noted that the pharmacokinetics of atezolizumab administered as a single agent have been characterized based on clinical data from study PCD4989g and are consistent with a currently ongoing Phase III Study WO29522 in first line treatment of TNBC. Atezolizumab anti-tumor activity has been observed across doses from 1 to 20 mg/kg. Overall, atezolizumab exhibits pharmacokinetics that are both linear and consistent with typical IgG1 antibodies for doses ≥ 1 mg/kg every three weeks (q3w). Pharmacokinetic data (Bai S, Jorga K, Xin Y, et al., A guide to rational dosing of monoclonal antibodies, Clin Pharmacokinet 2012;51:119-35 ) does not suggest any clinically meaningful differences in exposure following a fixed dose or a dose adjusted for weight. Atezolizumab dosing schedules of q3w and q2w have been tested. A fixed dose of atezolizumab 800 mg every two weeks (q2w) (equivalent to a body weight-based dose of 10 mg/kg q2w) results in equivalent exposure to the Phase III dose of 1200 mg administered every three weeks (q3w). The q3w schedule is being used in multiple Phase III studies of atezolizumab monotherapy across multiple tumor types and the q2w predominantly used in combination with chemotherapy regimens. In Study PCD4989g, the Kaplan-Meier estimated overall 24-week progression-free survival (PFS) rate was 33% (95% CI: 12%, 53%).

The PD-1 axis inhibitor doses of the present disclosure are suitably from 400 mg to 1200 mg, from 600 mg to 1000 mg, from 700 mg to 900 mg, or 840 mg. In some aspects, the PD-1 axis inhibitor is the PD-L1 inhibitor atezolizumab, which is administered at a dose of 840 mg.

In particular embodiments, the PD-1 axis inhibitor, or more particularly the PD-L1 inhibitor, is administered intravenously every 14 days of a 28-day treatment cycle. In some aspects, the subject is treated with the PD-1 axis inhibitor, and more particularly the PD-L1 inhibitor, on days 1 and 15 of the 28-day treatment cycle.

### VEGF inhibitors

The VEGF inhibitors of the present disclosure bevacizumab (Avastin^{®}).

Bevacizumab has been approved by the FDA for use in combination with a chemotherapy regimen to treat metastatic colorectal cancer (CRC) and non-small cell lung cancer (NSCLC). Bevacizumab is described in U.S. Patent Nos. 6,054,297 and 6,884,879 , and is a recombinant humanized IgG1 mAb that binds VEGF and neutralizes the biological activity of VEGF by preventing the interaction of VEGF with its receptors.

In this or another aspect of the disclosure, the VEGF inhibitor is an antibody comprising a heavy chain variable region comprising the heavy chain variable region amino acid sequence from SEQ ID NO:33, and/or a light chain variable region comprising the light chain variable region amino acid sequence from SEQ ID NO:34. In still a further aspect of the disclosure is provided an isolated VEGF inhibitor antibody comprising a heavy chain and/or a light chain sequence wherein:
(a) the heavy chain sequence has at least 85%, at least 90%, at least 91%, at least 92%, at least 93%, at least 94%, at least 95%, at least 96%, at least 97%, at least 98%, at least 99%, or 100%, sequence identify to the heavy chain sequence; or,
(b) the light chain sequence has at least 85%, at least 90%, at least 91%, at least 92%, at least 93%, at least 94%, at least 95%, at least 96%, at least 97%, at least 98%, at least 99%, or 100%, sequence identify to the heavy chain sequence;

In this or yet another aspect of the disclosure, the VEGF inhibitor is an antibody comprising a heavy chain and/or a light chain variable region sequence, wherein:
(a) the heavy chain further comprises an HVR-H1, an HVR-H2 and an HVR-H3 sequence having at least 85% sequence identity to GYTFTNYGMN (SEQ ID NO:35), WINTYTGEPTYAADFKR (SEQ ID NO:36), and YPHYYGSSHWYFDV (SEQ ID NO:37), respectively; or,
(b) the light chain further comprises an HVR-L1, and HVR-L2 and an HVR-L3 sequence having at least 85% sequence identity to SASQDISNYLN (SEQ ID NO:38), FTSSLHS (SEQ ID NO:39), and QQYSTVPWT (SEQ ID NO:40), respectively.

In accordance with the present disclosure, the VEGF inhibitor dose is from 1 to about 15 mg/kg/week, from 5 to 15 mg/kg/week, from 5 to 10 mg/kg/week, such as 5 mg/kg/week, 10 mg/kg/week or 15 mg/kg/week. In some aspects, bevacizumab is administered weekly, and more particularly is administered at a dose of 5 mg/kg/week.

### Colorectal Cancer

In one aspect, provided herein is a method for treating of colorectal cancer in a subject in need thereof comprising administering to the subject a therapeutically effective amount of a combination of a MEK inhibitor, a PD-1 axis inhibitor, and a VEGF inhibitor. mCRC is particularly amenable to the combination therapy described herein.

In some aspects of the disclosure, the treatment results in delaying the progression of the CRC in the subject. In some other aspects, the treatment results in a complete response in the subject. In some other aspects, the response is sustained after cessation of the treatment. In still other aspects, the treatment prolongs the median progression-free survival time as compared to a CRC subject receiving a therapy comprising (i) the therapeutically effective amount of the PD-1 axis inhibitor and the therapeutically effective amount of the MEK inhibitor and without administration of the VEGF inhibitor, (ii) the therapeutically effective amount the PD-1 axis inhibitor and the therapeutically effective amount of the VEGF inhibitor and without administration of the MEK inhibitor, and/or (iii) the therapeutically effective amount the MEK inhibitor and the therapeutically effective amount of the VEGF inhibitor and without administration of the PD-1 axis inhibitor.

### Combination Therapies

It is believed that the triple combination of a MEK inhibitor, a PD-1 axis inhibitor, and a VEGF inhibitor (i) targets the hallmarks of cancer (i.e., proliferative signaling, immune evasion, and angiogenesis), (ii) will lead to synergistic anti-tumor activity based upon the complex interplay and activity these agents exhibit, and/or (iii) will offer the potential for substantial clinical benefit in patients with CRC.

It is still further believed that, the triple combination treatments of the present disclosure may prolong the median progression-free survival time for a subject having CRC as compared to a subject having CRC receiving a therapy comprising (i) the therapeutically effective amount of the PD-1 axis inhibitor and the therapeutically effective amount of the MEK inhibitor and without administration of the VEGF inhibitor, (ii) the therapeutically effective amount the PD-1 axis inhibitor and the therapeutically effective amount of the VEGF inhibitor and without administration of the MEK inhibitor, and/or (iii) the therapeutically effective amount the MEK inhibitor and the therapeutically effective amount of the VEGF inhibitor and without administration of the PD-1 axis inhibitor.

### Drug Combination

In some aspects of the present disclosure, a cancer therapy drug combination is provided comprising: (i) a MEK inhibitor in a dose of from 20 mg to 100 mg, from 40 mg to 80 mg, or 80 mg; (ii) a PD-1 axis inhibitor in a dose of from 400 mg to 1200 mg, from 600 mg to 1000 mg, from 700 mg to 900 mg, or 840 mg; and (iii) a VEGF inhibitor in a dose from 5 mg/kg to 15 mg/kg, from 5 mg/kg to 10 mg/kg, 5 mg/kg, 10 mg/kg or 15 mg/kg. In the invention, the MEK inhibitor is cobimetinib, the PD-L1 inhibitor is atezolizumab, and the VEGF inhibitor is bevacizumab. In some aspects, the combination may be administered every two weeks. For instance, the combination may be administered on days 1 and 15 of a 28-day treatment cycle.

In this regard it is to be noted that any combination of the recited dosages ranges for a recited component of the combination may be used without departing from the intended scope of the present disclosure. When a subject is administered the drug combination (i.e., the MEK inhibitor, the PD-1 axis inhibitor and the VEGF inhibitor) on the same day, the drugs may be administered in any order. For instance, (i) the drugs may be administered separately in any order or (ii) a first drug and a second drug may be administered at the same time or closely spaced in time and a third drug may be administered either before or after administration of the first and second drug. Administration of each drug of the drug combination may be separated by some period of time, such as 0.5 hours, 1 hour, 2 hours, 3 hours or 4 hours. In some particular aspects, cobimetinib may be administered orally, atezolizumab may be administered intravenously, and bevacizumab may administered parentally or intravenously at least 0.5 hours after atezolizumab administration. In such aspects, cobimetinib may be administered before or after atezolizumab. In some aspects, the MEK inhibitor and the PD-1 axis inhibitor are each administered on days 1 and 15 of a 28-day treatment cycle, and cobimetinib is administered on days 1 to 21 of the 28-day treatment cycle.

### Kits

In some aspects of the disclosure, a kit for treating CRC in a human subject is provided. The kits comprise a MEK inhibitor, a PD-1 axis inhibitor, a VEGF inhibitor and a package insert comprising instructions for using a therapeutically effective amount of the MEK inhibitor, a therapeutically effective amount of the PD-1 axis inhibitor and a therapeutically the invention effective amount of the VEGF inhibitor for treating the subject. In the invention, the MEK inhibitor is cobimetinib, the PD-1 axis inhibitor is atezolizumab, and the VEGF inhibitor is bevacizumab.

The kits of the present disclosure prolongs the median progression-free survival time as compared to a CRC subject receiving a therapy comprising (i) the therapeutically effective amount of the PD-1 axis inhibitor and the therapeutically effective amount of the MEK inhibitor and without administration of the VEGF inhibitor, (ii) the therapeutically effective amount the PD-1 axis inhibitor and the therapeutically effective amount of the VEGF inhibitor and without administration of the MEK inhibitor, and/or (iii) the therapeutically effective amount the MEK inhibitor and the therapeutically effective amount of the VEGF inhibitor and without administration of the PD-1 axis inhibitor.

### Examples

The examples are directed to a two stage, open-label, multicenter, single-arm, Phase Ib, study designed to evaluate the safety, tolerability and pharmacokinetics of the combination of cobimetinib, atezolizumab and bevacizumab in patients with mCRC who have received and progressed on at least one prior line of therapy containing a fluoropyrimidine and oxaliplatin or irinotecan for advanced disease.

Stage 1 will be a safety run-in. Stage 2 will be a dose expansion with an expansion cohort and a biopsy cohort. Patients will first be accrued into the safety run-in phase. Upon determination of the safety and tolerability of the treatment regimen, the study will proceed to the expansion stage. If the results from the safety run-in stage require dose reduction in cobimetinib, then an additional Stage 1 cohort will be opened. Within the expansion stage patients can be enrolled either into the treatment or biopsy cohort depending on the suitability and willingness of the patient to undergo serial tumor biopsies. The study will end when all patients enrolled have been followed until death, withdrawal of consent, lost to follow-up, or the Sponsor decides to end the trial, whichever occurs first.

Primary objectives of the study include an assessment of assess the safety and tolerability of cobimetinib plus bevacizumab plus atezolizumab and confirmation of the proposed dosage regimen for further clinical development. Evaluation criteria and endpoints include: (i) the incidence, nature and severity of adverse events, graded according to the National Cancer Institute Common Terminology Criteria for Adverse Events (NCI CTCAE) v4; (ii) laboratory data; and (iii) Grade≥3 adverse events including adverse events of special interest, and adverse events leading to treatment discontinuations.

Exploratory efficacy objectives include and evaluation of the efficacy of cobimetinib plus bevacizumab plus atezolizumab. Evaluation criteria and endpoints include: (i) Investigator assessed confirmed overall response rate defined by Response Evaluation Criteria in Solid Tumors (RECIST) v1.1; (ii) progression-free survival, defined as the time from Cycle 1 Day 1 to the first occurrence of disease progression as determined by the investigator using RECIST v1.1 or death from any cause during the study, whichever occurs first; and (iii) duration of response, defined as the time from the first occurrence of a documented objective response to the time of disease progression as determined by the investigator using RECIST v1.1 or death from any cause during the study, whichever occurs first.

Study treatment will comprise cobimetinib at a dose of 60 mg on a 21/7 schedule, atezolizumab on an 840 mg every 2 week (q2w) schedule, and bevacizumab on a 5 mg/kg q2w schedule. Patients in the safety run-in and in the expansion cohorts will receive cobimetinib, atezolizumab, and bevacizumab from Day 1. Patients in the biopsy cohort in the expansion stage will start bevacizumab on Day 1 followed by a tumor biopsy on Day 14 (±2 day window) and the start of cobimetinib on Day 15 followed by a tumor biopsy on Day 28 (±2 day window) and the start of atezolizumab on Day 29 (i.e., Cycle 2 Day 1) followed by an optional tumor biopsy on Day 56 (±2 day window). Biopsies will be performed before the initiation of cobimetinib and atezolizumab respectively. From this point forward, patients in the biopsy cohort will follow the same treatment regimen as those in the safety run-in and treatment expansion cohorts.

The pretreatment biopsy will be required for all patients and will be formalin-fixed, paraffin embedded tissue. Archival biopsies can be used for the pretreatment biopsy as long as they are collected no more than 3 months prior to screening. Fresh samples are preferred for patients in the biopsy cohort. Remaining biopsies will be scheduled only for patients in the biopsy cohort as described above.

All patients will be closely monitored for safety and tolerability during all cycles of therapy, at the end-of-study treatment visit, and during the follow-up period. The NCI CTCAE v4.0 will be used to characterize the toxicity profile of the study treatments on all patients.

Patients in both stages will continue to receive study therapy until disease progression according to RECISTv1.1, unacceptable toxicity, death, patient or physician decision to withdraw, or pregnancy, whichever occurs first. Any evaluable and measurable disease will be documented at screening and re assessed at each subsequent tumor evaluation. Investigators will assess tumor response at 8 week intervals, regardless of any dose delays.

Treatment will continue until the patient has disease progression according to RECIST v1.1, unacceptable toxicity, death, patient or physician decision to withdraw, or pregnancy, whichever occurs first. A rising carcinoembryonic antigen level alone is will not be considered disease progression. Patients will be allowed to receive study treatment beyond disease progression if certain conditions are met.

Patient inclusion criteria for the study entry includes the following. At least 18 years of age. Eastern Cooperative Oncology Group performance status of 0 or 1. Histologically confirmed unresectable metastatic colorectal adenocarcinoma. Progression on a prior line of therapy containing a fluoropyrimidine and oxaliplatin or irinotecan for unresectable metastatic colorectal adenocarcinoma. Adjuvant or neoadjuvant chemotherapy is allowed, provided it is completed at least 12 months before start of study treatment. Measurable disease, according to RECIST v1.1. Note that lesions intended to be biopsied should not be target lesions. Adequate hematologic and end organ function, defined by the following laboratory results obtained within 14 days prior to first dose of study drug treatment: (i) WBC ≥ 2.5 and ≤ 15.0×10⁹/L; (ii) ANC ≥ 1.5×10⁹/L; (iii) Platelet count ≥ 100×10⁹/L; (iv) Hemoglobin ≥ 9 g/dL; (v) Albumin ≥ 9 g/dL; Serum bilirubin ≤ 1.5 the upper limit of normal (ULN) (patients with known Gilbert's disease may have a bilirubin ≤ 3.0 × ULN); (vi) INR and PTT ≤ 1.5 × ULN; (vii) amylase and lipase ≤ 1.5 × ULN; (viii) AST, ALT, and alkaline phosphatase (ALP) ≤ 3 × ULN with the following exceptions: Patients with documented liver metastases (AST and/or ALT ≤ 5 × ULN) and patients with documented liver or bone metastases (ALP ≤ 5 × ULN); (ix) creatine clearance ≥ 30 mL/min.

Patient exclusion criteria for the study entry includes the following. Surgical procedure (including open biopsy, surgical resection, wound revision or any other major surgery) or significant traumatic injury within 60 days prior to enrollment, or anticipation of need for major surgical procedure during the course of the study. Minor surgical procedure within 15 days (including placement of a vascular access device) of study Cycle 1 Day 1. Untreated CNS metastases. Treatment of brain metastases, either by surgical or radiation techniques, will have been completed at least 4 weeks prior to initiation of study treatment. Treatment with any investigational agent or approved therapy within 28 days or two investigational agent half-lives (whichever is longer) prior to enrollment in this study (Cycle 1 Day 1). Malignancies other than colorectal cancer within 5 years prior to Cycle 1 Day 1 with the exception of those with a negligible risk of metastasis or death (e.g., expected 5-year overall survival > 90%) treated with expected curative outcome (such as adequately treated carcinoma in situ of the cervix, basal or squamous cell skin cancer, localized prostate cancer treated surgically with curative intent, ducal carcinoma in situ treated surgically with curative intent. Prior radiation therapy within 30 days prior to study Cycle 1 Day 1 and/or persistence of radiation-related adverse effect. Prior allogeneic bone marrow transplantation or solid organ transplant for another malignancy in the past. Spinal cord compression not definitively treated with surgery and/or radiation. Uncontrolled pleural effusion, pericardial effusion, or ascites requiring recurrent drainage procedure.

Patient exclusion criteria for the study entry related to study medication includes the following. Current or recent (within 10 days of study enrollment) use of acetylsalicylic acid (>325 mg/day), clopidogrel (>75 mg/day) or current or recent (within 10 days of first dose of bevacizumab) use of therapeutic oral or parenteral anticoagulants or thrombolytic agents for therapeutic purpose. History of severe allergic, anaphylactic, or other hypersensitivity reactions to chimeric or humanized antibodies or fusion proteins. Known hypersensitivity or allergy to biopharmaceuticals produced in Chinese hamster ovary cells or any components of cobimetinib, atezolizumab, or bevacizumab formulation. Prior treatment with CD137 agonists or immune checkpoint blockage therapies, anti-programmed death-1, anti-program death-ligand 1, MEK inhibitor.

Patient exclusion criteria for the study entry related to organ function and medical history includes the following. History of clinically significant cardiac or pulmonary dysfunction. Serious non-healing wound, active ulcer or untreated bone fracture. History of abdominal fistula or gastrointestinal perforation within 6 months prior to Cycle 1 Day 1. History of hemoptysis (≥ ½ teaspoon of bright red blood per episode), or any other serious hemorrhage or at risk of bleeding (gastrointestinal history of bleeds, gastrointestinal ulcers, etc.). INR>1.5 and aPTT> 1.5×ULN within 7 days prior to Cycle 1 Day 1. History or evidence of inherited bleeding diathesis or significant coagulopathy at risk of bleeding. Life expectancy of < 12 weeks. Any previous venous thromboembolism ≥ Grade 3. Proteinuria at screening as demonstrated by urine dipstick ≥ 2+ or 24-hour proteinuria > 1.0 g. Left ventricular ejection fraction below institutional lower limit of normal. Uncontrolled serious medical or psychiatric illness. Uncontrolled tumor pain; patients who require narcotic pain medication during screening should be on a stable dose regimen prior to Cycle 1 Day 1. Pregnant or lactating, or intending to become pregnant during the study. Women who are not post-menopausal (≥ 12 continuous months of amenorrhea with no identified cause other than menopause) or surgically sterile must have a negative serum pregnancy test within 14 days prior to Cycle 1 Day 1. History of idiopathic pulmonary fibrosis, organizing pneumonia (e.g., bronchiolitis obliterans), drug-induced pneumonitis, idiopathic pneumonitis, or evidence of active pneumonitis on screening chest CT scan. History or evidence of retinal pathology on ophthalmologic examination that is considered a risk factor for neurosensory retinal detachment/central serous chorioretinopathy, retinal vein occlusion or neovascular macular degeneration. Exclusion criteria based on infectious diseases include: Active infection requiring IV antibiotics at screening; Patients with active hepatitis B (chronic or acute); Patients with past hepatitis B virus (HBV) infection or resolved HBV infection; Patients with active hepatitis C; and known HIV infection. Exclusion criteria based on autoimmune conditions including: history of autoimmune disease including but not limited to myasthenia gravis, myositis, autoimmune hepatitis, systemic lupus erythematosus, rheumatoid arthritis, inflammatory bowel disease, vascular thrombosis associated with antiphospholipid syndrome, Wegener's granulomatosis, Sjögren's syndrome, Guillain-Barre syndrome, multiple sclerosis, vasculitis, or glomerulonephritis.

Patient inclusion criteria for inclusion in the biopsy cohort includes: meeting all of the inclusion criteria for study entry; and bevacizumab naive or received the last bevacizumab treatment at least 12 months prior to Cycle 1 Day 1.

Cobimetinib will be administered at a dose of 60 mg cobimetinib (three tablets of 20 mg each) orally once daily for Days 1-21 of a 28-day cycle.

Atezolizumab will be administered at a dose of 840 mg by IV infusion on days 1 and 15 of each 28-day cycle. Atezolizumab will be administered first, followed by bevacizumab, with a minimum of 30 minutes between dosing.

Bevacizumab will be administered at a dose of 5 mg/kg by IV infusion on days 1 and 15 of each 28-day cycle.

### Collection of Archival and/or Fresh Tumor Specimens, and Biomarker Assessments

Extended RAS status has implications in treatment and prognosis in mCRC (Karapetis CS, Khambata-Ford S, Jonker DJ, et al., K-ras Mutations and Benefit from Cetuximab in Advanced Colorectal Cancer, N Engl J Med 2008;359:1757-65; De Roock W, Claes B, Bernasoni D, et al., Effects ofKRAS, BRAF, NRAS, and PIK3CA mutations on the efficacy of cetuximab plus chemotherapy in chemotherapy refractory metastatic colorectal cancer: a restrospective consortium analysis, The Lancet 2010;11:753-62; Sorich MJ, Wise MD, Rowland A, et al., Extended RAS mutations and anti-EGFR monocloncal antibody survival benefit in metastatic colorectal cancer: a meta analysis of randomized controlled trials, Ann Oncol 2015;26:13-21; and Allegra CJ, Rumble RB, Hamilton SR, et al., Extended RAS Gene Mutation Testing in Metastatic Colorectal Carcinoma to Predict Response to Anti-Epidermal Growth Factor Receptor Monoclonal Antibody Therapy: American Society of Clinical Oncology Provisional Clinical Opinion Update 2015, J Clin Oncol 2016;34:179-85. Each reference is incorporated by reference herein in its entirety.). Several studies demonstrate that RAS mutations carry a worse prognosis for PFS and overall survival compared to RAS wild type cohorts (Sorbye H, Dragomir A, Sundström M, et al., High BRAF Mutation Frequency and Marked Survival Differences in Subgroups According to KRAS/BRAFMutation Status and Tumor Tissue Availability in a Prospective Population-Based Metastatic Colorectal Cancer Cohort, PLoS One 2015;10:e0131046; Sorich et al. 2015; and Vincenzi B, Cremolini C, Sartore-Bianchi A, et al., Prognostic significance of K-Ras mutation rate in metastatic colorectal cancer patients, Oncotarget 2015;6:31604-12). The Phase Ib study GP28363 (cobimetinib administered with atezolizumab in patients with locally advanced or metastatic tumors) assessed safety and efficacy in KRAS mutant mCRC as well as in a biopsy cohort that included a variety of solid tumors. In connection with the present disclosure, the efficacy and safety of this regimen in all mCRC patients is evaluated regardless of extended RAS status because the mechanism of action would not predict a differential effect. However, given the differential prognosis based on RAS status, testing of archival/baseline tumor tissue for extended RAS status will be done.

MSI status in CRC also has implications in both the treatment and prognosis in mCRC (Goldstein J, Tran B, Ensor J, et al., Multicenter retrospective analysis of metastatic colorectal cancer (CRC) with high-level microsatellite instability (MSI-H), Annals of Oncology 2014;25:1032-8, incorporated by reference herein in its entirety). It has a different ORR and duration of response (DOR) rate than checkpoint inhibitors, such as PD L1 and PD 1 antagonists (Li J, Qin S, Xu R, et al., Regorafenib plus best supportive care versus placebo plus best supportive care in Asian patients with previously treated metastatic colorectal cancer (CONCUR): a randomised, double-blind, placebo-controlled, phase 3 trial, Lancet Oncol 2015;16:619-29; and Oh DY, Venook AP, Fong L., On the Verge: Immunotherapy for Colorectal Carcinoma, J Natl Comp Canc Netw 2015;13:970-8). It is believed that atezolizumab, a PD L1 antibody, may be effective in MSI high CRC patients, similar to other checkpoint inhibitors, but there may be a differential effect depending on MSI status. Thus, MSI status will be assessed from archival/baseline tissue to distinguish the efficacy of this regimen in these different populations.

Tumor tissue samples will be collected at baseline for DNA and/or RNA extraction to enable next generation sequencing (NGS) to identify somatic mutations to add to researchers' understanding of disease pathobiology. Gene-based CRC classification increasingly has been proposed as a way of differentiating various subtypes of CRC and may have profound effects on both treatment and prognosis. These subtypes have been shown to have different immunomodulatory affects and may influence the efficacy of this regimen (Guinney J, Dienstmann R, Wang X, et al., The consensus molecular subtypes of colorectal cancer, Nat Med 2015;21:1350-6; Kocarnik JM, Shiovitz S, Phipps AI, Molecular phenotypes of colorectal cancer and potential clinical applications, Gastroenterol Rep (Oxf) 2015;3:269-76; and Lal N, Beggs AD, Willcox BE, Middleton GW, An immunogenomic stratification of colorectal cancer: Implications for development of targeted immunotherapy, Oncoimmunology 2015;4:e976052). As these biomarkers may also have prognostic value, their potential association with disease progression will also be explored. Archival/baseline tumor analysis and classification of different CRC subtypes will be performed to further assess this possible relationship.

A stand-alone cohort where tumor biopsies at pretreatment, on treatment with bevacizumab, in combination with cobimetinib, and in triple combination with atezolizumab are scheduled. Comparison of biomarkers among the biopsies on staggered treatments will further elucidate the possible mechanism of action of this combination. The biomarker analyses will focus on the evaluation of CD8-positive T cell infiltrate, PD L1 expression, and biomarkers of enhanced immune response, MAPK inhibition, and others involved in apoptosis or inflammation.

### Stage 1: Safety Run-In Cohort

The Phase I study GP28363 has explored escalating doses of cobimetinib administered with atezolizumab. Cobimetinib administered with atezolizumab has shown to be safe and tolerable. In this study bevacizumab is added to the regimen of cobimetinib with atezolizumab. While bevacizumab has been administered with atezolizumab before, because bevacizumab has not previously been administered to patients along with the cobimetinib and atezolizumab, this study will start with a safety run in cohort. Bevacizumab has some overlapping toxicities with cobimetinib and atezolizumab.

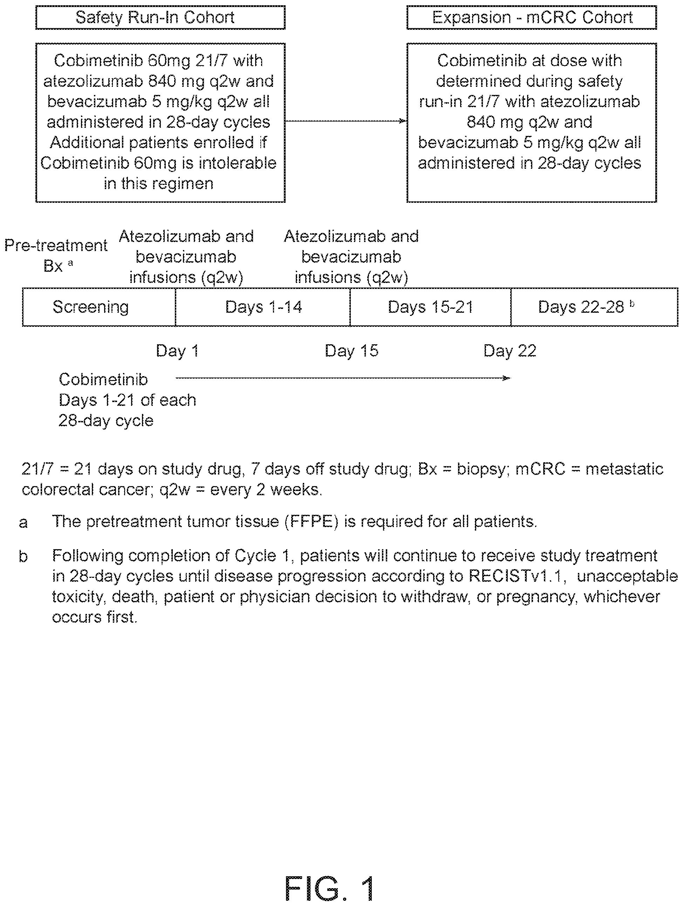

Patients in the safety run-in cohort will receive cobimetinib, atezolizumab, and bevacizumab from Day 1. A study schema is provided for the safety run-in and expansion cohorts is depicted in Figure 1.

In the safety run-in cohort, a 28-day dosing cycle will be evaluated where patients will be administered: (i) 60 mg cobimetinib for the first 21 days followed by a 7-day rest period without cobimetinib therapy; (ii) 840 mg atezolizumab infusion on days 1 and 15 of the 28-day cycle; and (iii) 5 mg/kg bevacizumab infusion on days 1 and 15 of the 28-day cycle.

After the patients in the safety run-in stage have completed at least one 28 day cycle of treatment, the clinical data will be reviewed to determine safety and tolerability of the tested doses. It is believed that it will take several months to complete enrolment of this group; therefore, the safety review should contain data for patients who have been receiving the regimen for several cycles at the time of the safety evaluation. If the 60 mg cobimetinib dose is determined to be not tolerable in the safety run-in stage, the study team may enroll an additional cohort of patients at a reduced cobimetinib dose of 40 mg QD (21/7). In such a reduced cobimetinib dosage evaluation, after the additional cohort has completed one cycle of treatment, a review of the clinical data will be conducted to determine if the expansion stage can initiate with this lower dose of cobimetinib.

If any of the following situations occur and assessed as related by the investigator, study treatment will be halted immediately for the individual patient, and a thorough investigation and safety analysis will be conducted: (i) Grade ≥ 3 hypertension (≥ 180 mmHg systolic or ≥ 110 mmHg diastolic; (ii) Grade ≥ 3 hemorrhage (symptoms and transfusion of ≤ 2 units packed RBCs indicated); (iii) Grade ≥ 3 pneumonitis (symptomatic, interfering with ADL; oxygen indicated); (iv) Grade ≥ 3 left ventricular dysfunction (symptomatic CHF responsive to intervention; (v) Grade ≥ 3 diarrhea not responsive to antidiarrheal agents (Increase of ≥7 stools per day over baseline; incontinence; IV fluids ≥24 hrs; hospitalization); or (vi) ALT or AST > 5 × ULN in combination with total bilirubin > 2 × ULN. If any of the following situations occur, then further enrollment and study treatments will be halted immediately until a thorough investigation and safety analysis has been conducted: (i) Any subject experiences death due to AE assessed as related to study treatment (by investigator and/or sponsor) will lead to temporary hold of study pending review by study team; or (ii) Over 30% of patients meet individual stopping rules defined above.

### Stage 2: Expansion and Biopsy Cohorts

Patients in the expansion cohort will receive cobimetinib, atezolizumab, and bevacizumab from Day 1.

Patients in the biopsy cohort in the expansion stage will start bevacizumab on Day 1 followed by a tumor biopsy on Day 14 (±2-day window) and the start of cobimetinib on Day 15 followed by a tumor biopsy on Day 28 (±2-day window) and the start of atezolizumab on Day 29 (i.e., Cycle 2 Day 1) followed by an optional tumor biopsy on Day 56 (±2-day window). Biopsies will be collected prior to the initiation of cobimetinib and atezolizumab, respectively. From this point forward, patients in the biopsy cohort will follow the same treatment regimen as those in the safety run-in and treatment expansion cohorts.

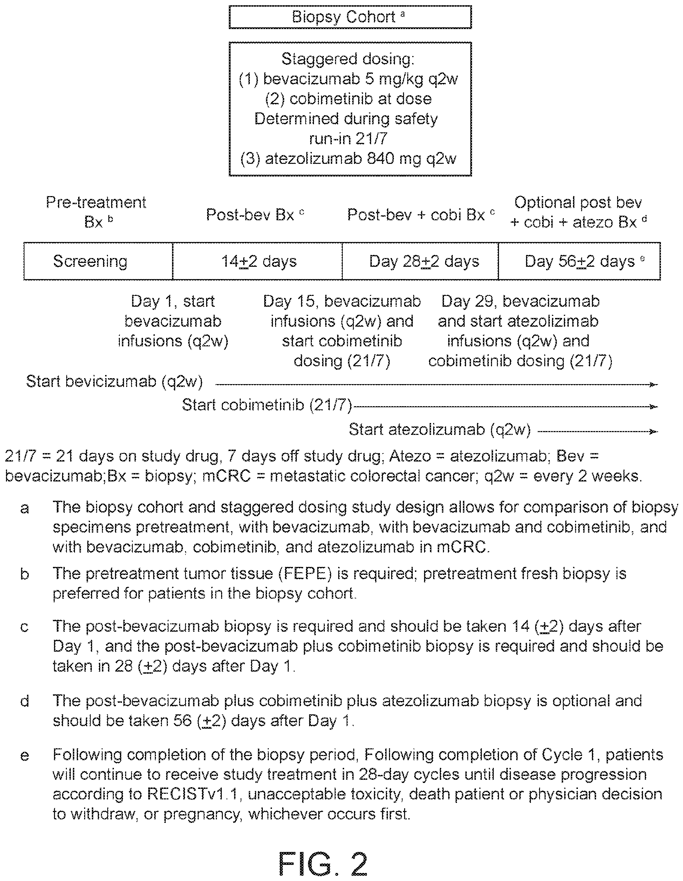

A study schema for the biopsy cohort is depicted in Figure 2.

Tumor response will be evaluated according to RECIST v1.1. Any evaluable and measurable disease will be documented at screening and reassessed at each subsequent tumor evaluation. Baseline tumor assessments will be performed ≤ 28 days before Cycle 1 Day 1 and assessed according to RECIST v 1.1 as outlined below. The same procedure used to assess disease sites at baseline will be used throughout the study (e.g., the same contrast protocol for CT scans or MRI scans).

Evaluation of tumor response conforming to RECIST v1.1 will be documented every 8 weeks ± 1 week (no matter where the patient is in the treatment cycle) until documented, investigator-determined, progressive disease, loss of clinical benefit, withdrawal of consent, death, or study termination by the Sponsor, whichever occurs first. Schedule of tumor assessments are independent of any changes to the study treatment administration schedule (e.g., dose delay) and may occur mid-cycle depending on length of cycle. If a tumor assessment has to be performed early or late, subsequent assessments should be conducted according to the original schedule based on the date of first study drug administration (Cycle 1, Day 1). Confirmation of response (PR or complete response [CR]) will be done no earlier than 28 days from study entry. In the case of SD, measurements must have met the SD criteria at least once after study entry at a minimum interval not less than 6 weeks. Patients who discontinue study treatment for any reason other than disease progression will continue to undergo tumor response evaluations (approximately every 8 weeks) until progressive disease. Rising tumor markers (e.g., CEA) in the absence of radiological evidence of progression is not considered progressive disease.

### Response evaluation in solid tumors

At baseline, tumor lesions/lymph nodes will be categorized measurable or non-measurable as follows. Tumor lesions will be measured in at least one dimension (longest diameter in the plane of measurement is to be recorded) with a minimum size of: (i) 10 mm by computed tomography (CT) or magnetic resonance imaging (MRI) scan (CT/MRI scan slice thickness/interval no greater than 5 mm); (ii) 10 mm caliper measurement by clinical examination (lesions that cannot be accurately measured with calipers should be recorded as non-measurable); or (iii) 20 mm by chest X-ray. Malignant lymph nodes will be considered pathologically enlarged and measurable is a lymph node is ≥ 15 mm in the short axis when assessed by CT scan (CT scan slice thickness recommended to be no greater than 5 mm). At baseline and in follow-up, only the short axis will be measured and followed. Non-measurable tumor lesions encompass small lesions (longest diameter < 10 mm or pathological lymph nodes with ≥ 10 to ≤ 15 mm short axis), as well as truly non measurable lesions. Lesions considered truly non-measurable include leptomeningeal disease, ascites, pleural or pericardial effusion, inflammatory breast disease, lymphangitic involvement of skin or lung, peritoneal spread, and abdominal masses/abdominal organomegaly identified by physical examination that is not measurable by reproducible imaging techniques.

In connection with bone lesions, bone scan, positron emission tomography (PET) scan, or plain films are not considered adequate imaging techniques to measure bone lesions. However, these techniques can be used to confirm the presence or disappearance of bone lesions. Lytic bone lesions or mixed lytic-blastic lesions, with identifiable soft tissue components, that can be evaluated by cross-sectional imaging techniques such as CT or MRI can be considered measurable lesions if the soft tissue component meets the definition of measurability described above. Blastic bone lesions are non-measurable. In connection with cystic lesions, lesions that meet the criteria for radiographically defined simple cysts should not be considered malignant lesions (neither measurable nor non-measurable) since they are, by definition, simple cysts. Cystic lesions thought to represent cystic metastases can be considered measurable lesions if they meet the definition of measurability described above. However, if noncystic lesions are present in the same patient, these are preferred for selection as target lesions. Tumor lesions situated in a previously irradiated area, or in an area subjected to other loco-regional therapy, are usually not considered measurable unless there has been demonstrated progression in the lesion. Study protocols should detail the conditions under which such lesions would be considered measurable.

Lesion measurements will be recorded in metric notation, using calipers if clinically assessed. All baseline evaluations will be performed as close as possible to the treatment start and never more than 4 weeks before the beginning of the treatment. The same method of assessment and the same technique will be used to characterize each identified and reported lesion at baseline and during study. Imaging based evaluation is preferred.

Clinical lesions will only be considered measurable when they are superficial and ≥ 10 mm in diameter as assessed using calipers (e.g., skin nodules). For the case of skin lesions, documentation by color photography, including a ruler to estimate the size of the lesion, is preferred.

Chest CT is preferred over chest X-ray, particularly when progression is an important endpoint, since CT is more sensitive than X-ray, particularly in identifying new lesions. However, lesions on chest X-ray may be considered measurable if they are clearly defined and surrounded by aerated lung. CT is the best currently available and reproducible method to measure lesions selected for response assessment. This guideline has defined measurability of lesions on CT scan based on the assumption that CT slice thickness is 5 mm or less. When CT scans have slice thickness greater than 5 mm, the minimum size for a measurable lesion should be twice the slice thickness. MRI is also acceptable. If, prior to enrollment, it is known that a patient is unable to undergo CT scans with intravenous (IV) contrast due to allergy or renal insufficiency, the decision as to whether a noncontrast CT or MRI (without IV contrast) will be used to evaluate the patient at baseline and during the study should be guided by the tumor type under investigation and the anatomic location of the disease. For patients who develop contraindications to contrast after baseline contrast CT is done, the decision as to whether non-contrast CT or MRI (enhanced or non-enhanced) will be performed should also be based on the tumor type and the anatomic location of the disease and should be optimized to allow for comparison with the prior studies if possible.

Assessment of tumor response involves an estimate of the overall tumor burden at baseline and to use this as a comparator for subsequent measurements. Measurable disease is defined by the presence of at least one measurable lesion, as detailed above. When more than one measurable lesion is present at baseline, all lesions up to a maximum of five lesions total (and a maximum of two lesions per organ) representative of all involved organs should be identified as target lesions and will be recorded and measured at baseline. This means in instances where patients have only one or two organ sites involved, a maximum of two lesions (one site) and four lesions (two sites), respectively, will be recorded. Other lesions (albeit measurable) in those organs will be recorded as non-measurable lesions (even if the size is > 10 mm by CT scan). Target lesions should be selected on the basis of their size (lesions with the longest diameter) and be representative of all involved organs, but additionally, should lend themselves to reproducible repeated measurements. Lymph nodes that have a short axis < 10 mm are considered nonpathological and should not be recorded or followed. A sum of the diameters (longest for non-nodal lesions, short axis for nodal lesions) for all target lesions will be calculated and reported as the baseline sum of diameters. If lymph nodes are to be included in the sum, then, as noted above, only the short axis is added into the sum. The baseline sum of diameters will be used as a reference to further characterize any objective tumor regression in the measurable dimension of the disease.

A summary of the overall response status calculation at each timepoint for patients who have measurable disease at baseline is provided in Table 1 below. A summary of the overall response calculation where a confirmation of response is required is provided in Table 2 below.

**Table 1: Timepoint response - Patients with target lesions (with or without nontarget lesions**

| Target Lesions | Nontarget Lesions | New Lesions | Overall Response |
|---|---|---|---|
| CR | CR | No | CR |
| CR | Non-CR/non-PD | No | PR |
| CR | Not evaluated | No | PR |
| PR | Non-PD or not all evaluated | No | PR |
| SD | Non-PD or not all evaluated | No | SD |
| Not all evaluated | Non-PD | No | Not evaluable |
| PD | Any | Yes or no | PD |
| Any | PD | Yes or no | PD |
| Any | Any | Yes | PD |

**Table 2: Best overall response when confirmation is required**

| Overall Response at First Timepoint | Overall Response at Subsequent Timepoint | Best Overall Response |
|---|---|---|
| CR | CR | CR |
| CR | PR | SD, PD or PR^{a} |
| CR | SD | SD, provided minimum duration for SD was met; otherwise, PD |
| CR | PD | SD, provided minimum duration for SD was met; otherwise, PD |
| CR | Not evaluable | SD, provided minimum duration for SD was met; otherwise, not evaluable |
| PR | CR | PR |
| PR | PR | PR |
| PR | SD | SD |
| PR | PD | SD, provided minimum duration for SD was met; otherwise, PD |
| PR | Not evaluable | SD, provided minimum duration for SD was met; otherwise, not evaluable |
| Not Evaluable | Not evaluable | Not evaluable |

^{a} If a CR is truly met at the first timepoint, any disease seen at a subsequent timepoint, even disease meeting PR criteria relative to baseline, qualifies as PD at that point (since disease must have reappeared after CR). Best response would depend on whether the minimum duration for SD was met. However, sometimes CR may be claimed when subsequent scans suggest small lesions were likely still present and in fact the patient had PR, not CR, at the first timepoint. Under these circumstances, the original CR should be changed to PR and the best response is PR

### Laboratory, Biomarker, and Other Biological Samples and Assessments

Samples for the following laboratory tests will be sent to one or several central laboratories for analysis. For patients who show evidence of immune mediated toxicity, additional samples may be collected and analyzed including: (i) Anti-nuclear antibody; (ii) Anti-double-stranded DNA; (iii) Circulating anti-neutrophil cytoplasmic antibody; and (iv) Perinuclear anti-neutrophil cytoplasmic antibody. Pharmacokinetic assays will include: (i) Serum samples assayed for atezolizumab and bevacizumab concentrations with use of a validated immunoassay per the schedule in Table 3 below; and (ii) Plasma samples for cobimetinib concentrations measured using validated liquid chromatography combined with tandem mass spectrometry method per the schedule in Table 3 below. Blood samples will be collected and analyzed for biomarkers, including, but not limited to, biomarkers that are related to CRC or tumor immune biology from all eligible patients according to the schedule in Table 3. The samples will be processed to obtain plasma for determination of blood-based proposed biomarkers listed in Table 4.

**Table 3: Schedule of Pharmacokinetic, Immunogenicity, and Biomarker Samples**

| Visit | Timepoint | Sample Type |
|---|---|---|
| Cycle 1 Day 1 | Prior to the first infusion | Atezolizumab PK and ATA (serum) Biomarker |
| | 30 (±10) minutes following the end of atezolizumab infusion | Atezolizumab PK (serum) |
| | 2-4 hours after the cobimetinib dose | Cobimetinib PK (plasma) |
| Cycle 1 Day 15 | Prior to cobimetinib dose | Cobimetinib PK (plasma) |
| Cycle 2 Day 1 | Prior to the first infusion | Biomarker |
| Cycles 2, 4, 8, and every 8 cycles thereafter | Prior to the first infusion | Atezolizumab PK and ATA (serum) |
| Cycle 3 Day 1 | Prior to the first infusion | Biomarker |
| Cycle 3 Day 15 | Prior to atezolizumab infusion | Atezolizumab PK and ATA (serum) Bevacizumab PK (serum) |
| | Prior to cobimetinib dose | Cobimetinib PK (plasma) |
| | 30 minutes following the end of atezolizumab infusion | Atezolizumab PK and ATA (serum) |
| | 2-4 hours after cobimetinib dose | Cobimetinib PK (plasma) |
| Cycle 6 Day 1 and every 6 cycles thereafter | Prior to the first infusion | Biomarker |
| Treatment discontinuation visit ^{a} | At visit | Atezolizumab PK and ATA (serum) Biomarker |

| | | |
|---|---|---|
| ^{a} Patients who discontinue study drug will return to the clinic for a treatment discontinuation visit 30 (± 7) days after the last dose of study drug. The visit at which response assessment shows progressive disease may be used as the treatment discontinuation visit. | | |

**Table 4: Proposed Biomarkers for Exploratory Research**

| Sample Type | Timing | Proposed Biomarkers |
|---|---|---|
| Plasma | Baseline and subsequent timepoints during treatment | Cytokines and other immune regulators |
| Circulating tumor DNA isolated from plasma | Baseline and subsequent timepoints during treatment | A panel of oncogenic mutations and overall mutation loads |
| Tumor tissues | Prior to study (archival) or baseline for all patients (fresh; preferred for patients in the biopsy cohort), on-treatment (biopsy cohort) | Immune cell infiltrates, and protein expression, including but not limited to PD L1 and major histocompatibility expression |
| DNA and RNA extracted from tumor tissues | Prior to study (archival) or baseline (fresh), on-treatment (biopsy cohort) | Microsatellite, a panel of oncogenic mutations and overall mutation loads. CRC subtyping signatures |

### Tumor Tissue Samples

Representative tumor specimens in paraffin blocks (preferred) or at least 20 serial cut, unstained slides with an associated pathology report will be submitted for determination of RAS status and MSI status.

Extended RAS mutation is defined as mutations occurring in KRAS and NRAS gene codons 12 and 13 of exon 2; 59 and 61 of exon 3; and 117 and 146 of exon 4 (Allegra et al. 2016). Local RAS testing results will be accepted with a copy of the results and interpretation as part of the screening process with a requirement for central confirmation.

MSI status can be defined by several methods such as IHC detection of hMLH1 and hMSH2 gene products, NGS testing, or PCR testing by the fraction of MSI loci that exhibit differently sized repeats (Lindor NM, Burgart LJ, Leontovich O, et al., Immunohistochemistry versus microsatellite instability testing in phenotyping colorectal tumors, J Clin Oncol 2002;20:1043-8; Salipante SJ, Scroggins SM, Hampel HL, et al., Microsatellite instability detection by next generation sequencing., Clin Chem 2014;60:1192-9.). Local MSI testing results will be accepted with a copy of the results and interpretation as part of the screening process with a requirement for central confirmation.

In addition, NGS for exploratory research on non-inherited (or tumor specific) biomarkers (including, but not limited to, cancer-related genes and biomarkers associated with common molecular pathways) and exploratory biomarkers (including but not limited to markers related to immune, MAP kinase pathway, or CRC biology, such as T-cell markers or tumor mutation status) may be evaluated.

## Claims

1. A drug combination for use in treating a subject having colorectal cancer, the drug combination being administered to said subject and comprising (i) a therapeutically effective amount of the MEK inhibitor cobimetinib or a pharmaceutically acceptable salt thereof, (ii) a therapeutically effective amount of the PD-1 axis inhibitor atezolizumab, and (iii) a therapeutically effective amount of the VEGF inhibitor bevacizumab, and further wherein the subject is to be treated with from 20 mg to 100 mg of cobimetinib or a pharmaceutically acceptable salt thereof per day, with from 400 mg to 1200 mg of atezolizumab intravenously every 14 days of a 28-day treatment cycle, and with from 3 mg per kg body weight to 7 mg per kg body weight of bevacizumab every 14 days of a 28-day treatment cycle.

2. The drug combination for use of claim 1 wherein the subject is treated with: 60 mg of cobimetinib or a pharmaceutically acceptable salt thereof; 840 mg of atezolizumab; and 5 mg per kg body weight of bevacizumab.

3. The drug combination for use of claim 1 or claim 2, wherein cobimetinib or a pharmaceutically acceptable salt thereof is administered once daily for 21 consecutive days of a 28-day treatment cycle, atezolizumab is administered on days 1 and 15 of the 28-day treatment cycle, and bevacizumab is administered on days 1 and 15 of the 28-day treatment cycle.

4. The drug combination for use of any one of claims 1 to 3, wherein cobimetinib or a pharmaceutically acceptable salt thereof, atezolizumab and bevacizumab are each administered on day 1 and day 15 of a 28-day treatment cycle, and cobimetinib or a pharmaceutically acceptable salt thereof is administered on days 1 to 21 of the 28-day treatment cycle.

5. The drug combination for use of any one of claims 1 to 4, wherein the subject has metastatic colorectal cancer or microsatellite stable colorectal cancer.

6. A kit for use in treating colorectal cancer in a human subject, the kit comprising the MEK inhibitor cobimetinib or a pharmaceutically acceptable salt thereof, the PD-1 axis inhibitor atezolizumab, the VEGF inhibitor bevacizumab and a package insert comprising instructions for using a therapeutically effective amount of cobimetinib or a pharmaceutically acceptable salt thereof, a therapeutically effective amount of atezolizumab and a therapeutically effective amount of bevacizumab for treating the subject, and further wherein the subject is to be treated with:
from 20 mg to 100 mg of cobimetinib or a pharmaceutically acceptable salt thereof per day,
from 400 mg to 1200 mg of atezolizumab intravenously every 14 days of a 28-day treatment cycle, and
from 3 mg per kg body weight to 7 mg per kg body weight of bevacizumab every 14 days of a 28-day treatment cycle.

7. A drug combination for use in colorectal cancer therapy, the drug combination comprising:
(i) the MEK inhibitor cobimetinib or a pharmaceutically acceptable salt thereof in a dose of from 20 mg to 100 mg;
(ii) the PD-1 axis inhibitor atezolizumab in a dose of from 400 mg to 1200 mg; and
(iii) the VEGF inhibitor bevacizumab in a dose of from 5 mg/kg to 15 mg/kg.

8. The drug combination for use of claim 7 wherein the cobimetinib or a pharmaceutically acceptable salt thereof is in a dose of 60 mg, the atezolizumab is in a dose of 840 mg, and the bevacizumab is in a dose of 5 mg per kg body weight.

## Patentansprüche

1. Arzneimittelkombination zur Verwendung bei der Behandlung eines Individuums mit Kolorektalkrebs, wobei die Arzneimittelkombination dem Individuum verabreicht wird und (i) eine therapeutisch wirksame Menge des MEK-Inhibitors Cobimetinib oder ein pharmazeutisch annehmbares Salz davon, (ii) eine therapeutisch wirksame Menge des PD-1-Achse-Inhibitors Atezolizumab und (iii) eine therapeutisch wirksame Menge des VEGF-Inhibitors Bevacizumab umfasst und wobei das Individuum weiters mit 20 mg bis 100 mg Cobimetinib oder einem pharmazeutisch annehmbaren Salz davon pro Tag, mit 400 mg bis 1200 mg Atezolizumab intravenös alle 14 Tage über einen 28-tägigen Behandlungszyklus und mit 3 mg pro kg Körpergewicht bis 7 mg pro kg Körpergewicht Bevacizumab alle 14 Tage über einen 28-tägigen Behandlungszyklus behandelt wird.

2. Arzneimittelkombination zur Verwendung nach Anspruch 1, wobei das Individuum mit 60 mg Cobimetinib oder einem pharmazeutisch annehmbaren Salz davon; 840 mg Atezolizumab; und 5 mg pro kg Körpergewicht Bevacizumab behandelt wird.

3. Arzneimittelkombination zur Verwendung nach Anspruch 1 oder Anspruch 2, wobei Cobimetinib oder ein pharmazeutisch annehmbares Salz davon einmal täglich über 21 aufeinanderfolgende Tage eines 28-tägigen Behandlungszyklus verabreicht wird, Atezolizumab am Tag 1 und 15 des 28-tägigen Behandlungszyklus verabreicht wird und Bevacizumab am Tag 1 und 15 des 28-tägigen Behandlungszyklus verabreicht wird.

4. Arzneimittelkombination zur Verwendung nach einem der Ansprüche 1 bis 3, wobei Cobimetinib oder ein pharmazeutisch annehmbares Salz davon, Atezolizumab und Bevacizumab jeweils am Tag 1 und Tag 15 eines 28-tägigen Behandlungszyklus verabreicht werden und Cobimetinib oder ein pharmazeutisch annehmbares Salz davon an den Tagen 1 bis 21 des 28-tägigen Behandlungszyklus verabreicht wird.

5. Arzneimittelkombination zur Verwendung nach einem der Ansprüche 1 bis 4, wobei das Individuum metastatischen Kolorektalkrebs oder Mikrosatelliten-stabilen Kolorektalkrebs hat.

6. Set zur Verwendung bei der Behandlung von Kolorektalkrebs bei einem menschlichen Individuum, wobei das Set den MEK-Inhibitor Cobimetinib oder ein pharmazeutisch annehmbares Salz davon, den PD-1-Achse-Inhibitor Atezolizumab, den VEGF-Inhibitor Bevacizumab und eine Packungsbeilage mit Anweisungen zur Verwendung einer therapeutisch wirksamen Menge Cobimetinib oder eines pharmazeutisch annehmbaren Salzes davon, einer therapeutisch wirksamen Menge Atezolizumab und einer therapeutisch wirksamen Menge Bevacizumab zur Behandlung des Individuums umfasst und wobei das Individuum außerdem mit Folgendem behandelt wird:
20 mg bis 100 mg Cobimetinib oder einem pharmazeutisch annehmbaren Salz davon pro Tag,
400 mg bis 1200 mg Atezolizumab intravenös alle 14 Tage über einen 28-tägigen Behandlungszyklus und
3 mg pro kg Körpergewicht bis 7 mg pro kg Körpergewicht Bevacizumab alle 14 Tage über einen 28-tägigen Behandlungszyklus.

7. Arzneimittelkombination zur Verwendung bei einer Kolorektalkrebstherapie, wobei die Arzneimittelkombination Folgendes umfasst:
(i) den MEK-Inhibitor Cobimetinib oder ein pharmazeutisch annehmbares Salz davon in einem Dosis von 20 mg bis 100 mg;
(ii) den PD-1-Achse-Inhibitor Atezolizumab in einer Dosis von 400 mg bis 1200 mg; und
(iii) den VEGF-Inhibitor Bevacizumab in einer Dosis von 5 mg/kg bis 15 mg/kg.

8. Arzneimittelkombination zur Verwendung nach Anspruch 7, wobei das Cobimetinib oder ein pharmazeutisch annehmbares Salz davon in einer Dosis von 60 mg, das Atezolizumab in einer Dosis von 840 mg und das Bevacizumab in einer Dosis von 5 mg pro kg Körpergewicht vorhanden ist.

## Revendications

1. Combinaison médicamenteuse à utiliser dans le traitement d'un sujet présentant un cancer colorectal, la combinaison médicamenteuse étant administrée audit sujet et comprenant (i) une quantité thérapeutiquement efficace de l'inhibiteur de MEK cobimétinib ou d'un sel pharmaceutiquement acceptable de celui-ci, (ii) une quantité thérapeutiquement efficace de l'inhibiteur d'axe PD-1 atézolizumab, et (iii) une quantité thérapeutiquement efficace de l'inhibiteur de VEGF bévacizumab, et dans laquelle en outre le sujet doit être traité avec de 20 mg à 100 mg de cobimétinib ou d'un sel pharmaceutiquement acceptable de celui-ci par jour, avec de 400 mg à 1 200 mg d'atézolizumab par voie intraveineuse tous les 14 jours d'un cycle de traitement de 28 jours, et avec de 3 mg par kg de poids corporel à 7 mg par kg de poids corporel de bévacizumab tous les 14 jours d'un cycle de traitement de 28 jours.

2. Combinaison médicamenteuse à utiliser selon la revendication 1, dans laquelle le sujet est traité avec : 60 mg de cobimétinib ou d'un sel pharmaceutiquement acceptable de celui-ci ; 840 mg d'atézolizumab ; et 5 mg par kg de poids corporel de bévacizumab.

3. Combinaison médicamenteuse à utiliser selon la revendication 1 ou la revendication 2, dans laquelle le cobimétinib ou un sel pharmaceutiquement acceptable de celui-ci est administré une fois par jour pendant 21 jours consécutifs d'un cycle de traitement de 28 jours, l'atézolizumab est administré les jours 1 et 15 du cycle de traitement de 28 jours, et le bévacizumab est administré les jours 1 et 15 du cycle de traitement de 28 jours.

4. Combinaison médicamenteuse à utiliser selon l'une quelconque des revendications 1 à 3, dans laquelle le cobimétinib ou un sel pharmaceutiquement acceptable de celui-ci, l'atézolizumab et le bévacizumab sont chacun administrés les jours 1 et 15 d'un cycle de traitement de 28 jours, et le cobimétinib ou un sel pharmaceutiquement acceptable de celui-ci est administré les jours 1 à 21 du cycle de traitement de 28 jours.

5. Combinaison médicamenteuse à utiliser selon l'une quelconque des revendications 1 à 4, dans laquelle le sujet présente un cancer colorectal métastatique ou un cancer colorectal stable aux microsatellites.

6. Kit à utiliser dans le traitement du cancer colorectal chez un sujet humain, le kit comprenant l'inhibiteur de MEK cobimétinib ou un sel pharmaceutiquement acceptable de celui-ci, l'inhibiteur d'axe PD-1 atézolizumab, l'inhibiteur de VEGF bévacizumab et un insert de conditionnement comprenant des instructions pour utiliser une quantité thérapeutiquement efficace de cobimétinib ou d'un sel pharmaceutiquement acceptable de celui-ci, une quantité thérapeutiquement efficace d'atézolizumab et une quantité thérapeutiquement efficace de bévacizumab pour traiter le sujet, et dans lequel en outre le sujet doit être traité avec :
de 20 mg à 100 mg de cobimétinib ou d'un sel pharmaceutiquement acceptable de celui-ci par jour,
de 400 mg à 1 200 mg d'atézolizumab par voie intraveineuse tous les 14 jours d'un cycle de traitement de 28 jours, et
de 3 mg par kg de poids corporel à 7 mg par kg de poids corporel de bévacizumab tous les 14 jours d'un cycle de traitement de 28 jours.

7. Combinaison médicamenteuse à utiliser dans la thérapie du cancer colorectal, la combinaison médicamenteuse comprenant :
(i) l'inhibiteur de MEK cobimétinib ou un sel pharmaceutiquement acceptable de celui-ci à une dose comprise entre 20 mg et 100 mg ;
(ii) l'inhibiteur d'axe PD-1 atézolizumab à une dose comprise entre 400 mg à 1 200 mg ; et
iii) l'inhibiteur de VEGF bévacizumab à une dose comprise entre 5 mg/kg et 15 mg/kg.

8. Combinaison médicamenteuse à utiliser selon la revendication 7, dans laquelle le cobimétinib ou un sel pharmaceutiquement acceptable de celui-ci est à une dose de 60 mg, l'atézolizumab est à une dose de 840 mg, et le bévacizumab est à une dose de 5 mg par kg de poids corporel.


### SEQUENCE LISTING

<110> Genentech, Inc.
<120> COMBINATION THERAPY WITH A MEK INHIBITOR, A PD-1 AXIS INHIBITOR, AND A VEGF INHIBITOR
<130> P33774 (33988-58)
<150> US 62/374437
   <151> 2016-08-12
<160> 40
<170> PatentIn version 3.5
<210> 1
   <211> 440
   <212> PRT
   <213> Artificial Sequence
<220>
   <223> Synthetic Peptide
<400> 1
   Gln Val Gln Leu Val Glu Ser Gly Gly Gly Val Val Gln Pro Gly Arg
   1               5                   10                  15
   Ser Leu Arg Leu Asp Cys Lys Ala Ser Gly Ile Thr Phe Ser Asn Ser
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Lys Arg Tyr Tyr Ala Asp Ser Val
       50                  55                  60
   Lys Gly Arg Phe Thr Ile Ser Arg Asp Asn Ser Lys Asn Thr Leu Phe
   65                  70                  75                  80
   Leu Gln Met Asn Ser Leu Arg Ala Glu Asp Thr Ala Val Tyr Tyr Cys
                   85                  90                  95
   Ala Thr Asn Asp Asp Tyr Trp Gly Gln Gly Thr Leu Val Thr Val Ser
               100                 105                 110
   Ser Ala Ser Thr Lys Gly Pro Ser Val Phe Pro Leu Ala Pro Cys Ser
           115                 120                 125
   Arg Ser Thr Ser Glu Ser Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Lys
               180                 185                 190
   Thr Tyr Thr Cys Asn Val Asp His Lys Pro Ser Asn Thr Lys Val Asp
           195                 200                 205
   Lys Arg Val Glu Ser Lys Tyr Gly Pro Pro Cys Pro Pro Cys Pro Ala
       210                 215                 220
   Pro Glu Phe Leu Gly Gly Pro Ser Val Phe Leu Phe Pro Pro Lys Pro
   225                 230                 235                 240
   Lys Asp Thr Leu Met Ile Ser Arg Thr Pro Glu Val Thr Cys Val Val
                   245                 250                 255
   Val Asp Val Ser Gln Glu Asp Pro Glu Val Gln Phe Asn Trp Tyr Val
               260                 265                 270
   Asp Gly Val Glu Val His Asn Ala Lys Thr Lys Pro Arg Glu Glu Gln
           275                 280                 285
   Phe Asn Ser Thr Tyr Arg Val Val Ser Val Leu Thr Val Leu His Gln
       290                 295                 300
   Asp Trp Leu Asn Gly Lys Glu Tyr Lys Cys Lys Val Ser Asn Lys Gly
   305                 310                 315                 320
   Leu Pro Ser Ser Ile Glu Lys Thr Ile Ser Lys Ala Lys Gly Gln Pro
                   325                 330                 335
   Arg Glu Pro Gln Val Tyr Thr Leu Pro Pro Ser Gln Glu Glu Met Thr
               340                 345                 350
   Lys Asn Gln Val Ser Leu Thr Cys Leu Val Lys Gly Phe Tyr Pro Ser
           355                 360                 365
   Asp Ile Ala Val Glu Trp Glu Ser Asn Gly Gln Pro Glu Asn Asn Tyr
       370                 375                 380
   Lys Thr Thr Pro Pro Val Leu Asp Ser Asp Gly Ser Phe Phe Leu Tyr
   385                 390                 395                 400
   Ser Arg Leu Thr Val Asp Lys Ser Arg Trp Gln Glu Gly Asn Val Phe
                   405                 410                 415
   Ser Cys Ser Val Met His Glu Ala Leu His Asn His Tyr Thr Gln Lys
               420                 425                 430
   Ser Leu Ser Leu Ser Leu Gly Lys
           435                 440
<210> 2
   <211> 214
   <212> PRT
   <213> Artificial Sequence
<220>
   <223> Synthetic Peptide
<400> 2
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Ser Ser Asn Trp Pro Arg
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 447
   <212> PRT
   <213> Artificial Sequence
<220>
   <223> Synthetic Peptide
<400> 3
   Gln Val Gln Leu Val Gln Ser Gly Val Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Tyr Met Tyr Trp Val Arg Gln Ala Pro Gly Gln Gly Leu Glu Trp Met
           35                  40                  45
   Gly Gly Ile Asn Pro Ser Asn Gly Gly Thr Asn Phe Asn Glu Lys Phe
       50                  55                  60
   Lys Asn Arg Val Thr Leu Thr Thr Asp Ser Ser Thr Thr Thr Ala Tyr
   65                  70                  75                  80
   Met Glu Leu Lys Ser Leu Gln Phe Asp Asp Thr Ala Val Tyr Tyr Cys
                   85                  90                  95
   Ala Arg Arg Asp Tyr Arg Phe Asp Met Gly Phe Asp Tyr Trp Gly Gln
               100                 105                 110
   Gly Thr Thr Val Thr Val Ser Ser Ala Ser Thr Lys Gly Pro Ser Val
           115                 120                 125
   Phe Pro Leu Ala Pro Cys Ser Arg Ser Thr Ser Glu Ser Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Lys Thr Tyr Thr Cys Asn Val Asp His Lys
           195                 200                 205
   Pro Ser Asn Thr Lys Val Asp Lys Arg Val Glu Ser Lys Tyr Gly Pro
       210                 215                 220
   Pro Cys Pro Pro Cys Pro Ala Pro Glu Phe Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser Gln Glu Asp Pro Glu
               260                 265                 270
   Val Gln Phe Asn Trp Tyr Val Asp Gly Val Glu Val His Asn Ala Lys
           275                 280                 285
   Thr Lys Pro Arg Glu Glu Gln Phe Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Gly Leu Pro Ser Ser Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Gln Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Arg Leu Thr Val Asp Lys Ser Arg
                       405                 410                 415
   Trp Gln Glu Gly Asn Val Phe Ser Cys Ser Val Met His Glu Ala Leu
               420                 425                 430
   His Asn His Tyr Thr Gln Lys Ser Leu Ser Leu Ser Leu Gly Lys
           435                 440                 445
<210> 4
   <211> 218
   <212> PRT
   <213> Artificial Sequence
<220>
   <223> Synthetic Peptide
<400> 4
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Lys Gly Val Ser Thr Ser
               20                  25                  30
   Gly Tyr Ser Tyr Leu His Trp Tyr Gln Gln Lys Pro Gly Gln Ala Pro
           35                  40                  45
   Arg Leu Leu Ile Tyr Leu Ala Ser Tyr Leu Glu Ser Gly Val Pro Ala
       50                  55                  60
   Arg Phe Ser Gly Ser Gly Ser Gly Thr Asp Phe Thr Leu Thr Ile Ser
   65                  70                  75                  80
   Ser Leu Glu Pro Glu Asp Phe Ala Val Tyr Tyr Cys Gln His Ser Arg
                   85                  90                  95
   Asp Leu Pro Leu Thr Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 5
   <211> 117
   <212> PRT
   <213> Artificial Sequence
<220>
   <223> Synthetic Peptide
<400> 5
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ser
               20                  25                  30
   Trp Ile His Trp Val Arg Gln Ala Pro Gly Lys Gly Leu Glu Trp Val
           35                  40                  45
   Ala Trp Ile Ser Pro Tyr Gly Gly Ser Thr Tyr Tyr Ala Asp Ser Val
       50                  55                  60
   Lys Gly Arg Phe Thr Ser Ala Asp Thr Ser Lys Asn Thr Ala Tyr Leu
   65                  70                  75                  80
   Gln Met Asn Ser Leu Arg Ala Glu Asp Thr Ala Val Tyr Tyr Cys Ala
                   85                  90                  95
   Arg Arg His Trp Pro Gly Gly Phe Asp Tyr Trp Gly Gln Gly Thr Leu
               100                 105                 110
   Val Thr Val Ser Ala
           115
<210> 6
   <211> 108
   <212> PRT
   <213> Artificial Sequence
<220>
   <223> Synthetic Peptide
<400> 6
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Leu Tyr His Pro Ala
                   85                  90                  95
   Thr Phe Gly Gln Gly Thr Lys Val Glu Ile Lys Arg
               100                 105
<210> 7
   <211> 118
   <212> PRT
   <213> Artificial Sequence
<220>
   <223> Synthetic Peptide
<400> 7
<210> 8
   <211> 122
   <212> PRT
   <213> Artificial Sequence
<220>
   <223> Synthetic Peptide
<400> 8
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asp Ser
   20 25 30
   Trp Ile His Trp Val Arg Gln Ala Pro Gly Lys Gly Leu Glu Trp Val
   35 40 45
   Ala Trp Ile Ser Pro Tyr Gly Gly Ser Thr Tyr Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Arg His Trp Pro Gly Gly Phe Asp Tyr Trp Gly Gln Gly Thr
   100 105 110
   Leu Val Thr Val Ser Ser Ala Ser Thr Lys
   115 120
<210> 9
   <211> 108
   <212> PRT
   <213> Artificial Sequence
<220>
   <223> Synthetic Peptide
<400> 9
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Leu Tyr His Pro Ala
                   85                  90                  95
   Thr Phe Gly Gln Gly Thr Lys Val Glu Ile Lys Arg
               100                 105
<210> 10
   <211> 10
   <212> PRT
   <213> Artificial Sequence
<220>
   <223> Synthetic Peptide
<220>
   <221> MOD_RES
   <222> (6)..(6)
   <223> Asp or Gly
<400> 10
   Gly Phe Thr Phe Ser Xaa Ser Trp Ile His
   1               5                   10
<210> 11
   <211> 18
   <212> PRT
   <213> Artificial Sequence
<220>
   <223> Synthetic Peptide
<220>
   <221> MOD_RES
   <222> (4)..(4)
   <223> Ser or Leu
<220>
   <221> MOD_RES
   <222> (10)..(10)
   <223> Thr or Ser
<400> 11
   Ala Trp Ile Xaa Pro Tyr Gly Gly Ser Xaa Tyr Tyr Ala Asp Ser Val
   1               5                   10                  15
   Lys Gly
<210> 12
   <211> 9
   <212> PRT
   <213> Artificial Sequence
<220>
   <223> Synthetic Peptide
<400> 12
   Arg His Trp Pro Gly Gly Phe Asp Tyr
   1               5
<210> 13
   <211> 25
   <212> PRT
   <213> Artificial Sequence
<220>
   <223> Synthetic Peptide
<400> 13
<210> 14
   <211> 13
   <212> PRT
   <213> Artificial Sequence
<220>
   <223> Synthetic Peptide
<400> 14
   Trp Val Arg Gln Ala Pro Gly Lys Gly Leu Glu Trp Val
   1               5                   10
<210> 15
   <211> 32
   <212> PRT
   <213> Artificial Sequence
<220>
   <223> Synthetic Peptide
<400> 15
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Ala Arg
               20                  25                  30
<210> 16
   <211> 11
   <212> PRT
   <213> Artificial Sequence
<220>
   <223> Synthetic Peptide
<400> 16
   Trp Gly Gln Gly Thr Leu Val Thr Val Ser Ala
   1               5                   10
<210> 17
   <211> 11
   <212> PRT
   <213> Artificial Sequence
<220>
   <223> Synthetic Peptide
<220>
   <221> MOD_RES
   <222> (5)..(5)
   <223> Asp or Val
<220>
   <221> MOD_RES
   <222> (6)..(6)
   <223> Val or Ile
<220>
   <221> MOD_RES
   <222> (7)..(7)
   <223> Ser or Asn
<220>
   <221> MOD_RES
   <222> (9)..(9)
   <223> Ala or Phe
<220>
   <221> MOD_RES
   <222> (10)..(10)
   <223> Val or Leu
<400> 17
   Arg Ala Ser Gln Xaa Xaa Xaa Thr Xaa Xaa Ala
   1 5 10
<210> 18
   <211> 7
   <212> PRT
   <213> Artificial Sequence
<220>
   <223> Synthetic Peptide
<220>
   <221> MOD_RES
   <222> (4)..(4)
   <223> Phe or Thr
<220>
   <221> MOD_RES
   <222> (6)..(6)
   <223> Tyr or Ala
<400> 18
   Ser Ala Ser Xaa Leu Xaa Ser
   1 5
<210> 19
   <211> 9
   <212> PRT
   <213> Artificial Sequence
<220>
   <223> Synthetic Peptide
<220>
   <221> MOD_RES
   <222> (3)..(3)
   <223> Tyr, Gly, Phe, or Ser
<220>
   <221> MOD_RES
   <222> (4)..(4)
   <223> Leu, Tyr, Phe or Trp
<220>
   <221> MOD_RES
   <222> (5)..(5)
   <223> Tyr, Asn, Ala, Thr, Gly, Phe, or Ile
<220>
   <221> MOD_RES
   <222> (6)..(6)
   <223> His, Val, Pro, Thr, or Ile
<220>
   <221> MOD_RES
   <222> (8)..(8)
   <223> Ala, Trp, Arg, Pro, or Thr
<400> 19
   Gln Gln Xaa Xaa Xaa Xaa Pro Xaa Thr
   1               5
<210> 20
   <211> 23
   <212> PRT
   <213> Artificial Sequence
<220>
   <223> Synthetic Peptide
<400> 20
   Asp Ile Gln Met Thr Gln Ser Pro Ser Ser Leu Ser Ala Ser Val Gly
   1               5                   10                  15
   Asp Arg Val Thr Ile Thr Cys
               20
<210> 21
   <211> 15
   <212> PRT
   <213> Artificial Sequence
<220>
   <223> Synthetic Peptide
<400> 21
   Trp Tyr Gln Gln Lys Pro Gly Lys Ala Pro Lys Leu Leu Ile Tyr
   1               5                   10                  15
<210> 22
   <211> 32
   <212> PRT
   <213> Artificial Sequence
<220>
   <223> Synthetic Peptide
<400> 22
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 23
   <211> 11
   <212> PRT
   <213> Artificial Sequence
<220>
   <223> Synthetic Peptide
<400> 23
   Phe Gly Gln Gly Thr Lys Val Glu Ile Lys Arg
   1               5                   10
<210> 24
   <211> 10
   <212> PRT
   <213> Artificial Sequence
<220>
   <223> Synthetic Peptide
<400> 24
   Gly Phe Thr Phe Ser Asp Ser Trp Ile His
   1               5                   10
<210> 25
   <211> 18
   <212> PRT
   <213> Artificial Sequence
<220>
   <223> Synthetic Peptide
<400> 25
   Ala Trp Ile Ser Pro Tyr Gly Gly Ser Thr Tyr Tyr Ala Asp Ser Val
   1               5                   10                  15
   Lys Gly
<210> 26
   <211> 11
   <212> PRT
   <213> Artificial Sequence
<220>
   <223> Synthetic Peptide
<400> 26
   Arg Ala Ser Gln Asp Val Ser Thr Ala Val Ala
   1               5                   10
<210> 27
   <211> 7
   <212> PRT
   <213> Artificial Sequence
<220>
   <223> Synthetic Peptide
<400> 27
   Ser Ala Ser Phe Leu Tyr Ser
   1               5
<210> 28
   <211> 9
   <212> PRT
   <213> Artificial Sequence
<220>
   <223> Synthetic Peptide
<400> 28
   Gln Gln Tyr Leu Tyr His Pro Ala Thr
   1               5
<210> 29
   <211> 118
   <212> PRT
   <213> Artificial Sequence
<220>
   <223> Synthetic Peptide
<400> 29
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ser
               20                  25                  30
   Trp Ile His Trp Val Arg Gln Ala Pro Gly Lys Gly Leu Glu Trp Val
           35                  40                  45
   Ala Trp Ile Ser Pro Tyr Gly Gly Ser Thr Tyr Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Arg His Trp Pro Gly Gly Phe Asp Tyr Trp Gly Gln Gly Thr
               100                 105                 110
   Leu Val Thr Val Ser Ala
           115
<210> 30
   <211> 11
   <212> PRT
   <213> Artificial Sequence
<220>
   <223> Synthetic Peptide
<400> 30
   Trp Gly Gln Gly Thr Leu Val Thr Val Ser Ser
   1               5                   10
<210> 31
   <211> 447
   <212> PRT
   <213> Artificial Sequence
<220>
   <223> Synthetic Peptide
<400> 31
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ser
               20                  25                  30
   Trp Ile His Trp Val Arg Gln Ala Pro Gly Lys Gly Leu Glu Trp Val
           35                  40                  45
   Ala Trp Ile Ser Pro Tyr Gly Gly Ser Thr Tyr Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Arg His Trp Pro Gly Gly Phe Asp Tyr Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Gln Thr Tyr Ile Cys Asn Val Asn His Lys Pro Ser
   195 200 205
   Asn Thr Lys Val Asp Lys Lys Val Glu Pro Lys Ser Cys Asp Lys Thr
   210 215 220
   His Thr Cys Pro Pro Cys Pro Ala Pro Glu Leu Leu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Ala Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Ser Arg Glu Glu Met Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly
   435 440 445
<210> 32
   <211> 214
   <212> PRT
   <213> Artificial Sequence
<220>
   <223> Synthetic Peptide
<400> 32
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Leu Tyr His Pro Ala
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 33
   <211> 123
   <212> PRT
   <213> Artificial Sequence
<220>
   <223> Synthetic Peptide
<400> 33
<210> 34
   <211> 108
   <212> PRT
   <213> Artificial Sequence
<220>
   <223> Synthetic Peptide
<400> 34
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg
               100                 105
<210> 35
   <211> 10
   <212> PRT
   <213> Artificial Sequence
<220>
   <223> Synthetic Peptide
<400> 35
   Gly Tyr Thr Phe Thr Asn Tyr Gly Met Asn
   1               5                   10
<210> 36
   <211> 17
   <212> PRT
   <213> Artificial Sequence
<220>
   <223> Synthetic Peptide
<400> 36
   Trp Ile Asn Thr Tyr Thr Gly Glu Pro Thr Tyr Ala Ala Asp Phe Lys
   1               5                   10                  15
   Arg
<210> 37
   <211> 14
   <212> PRT
   <213> Artificial Sequence
<220>
   <223> Synthetic Peptide
<400> 37
   Tyr Pro His Tyr Tyr Gly Ser Ser His Trp Tyr Phe Asp Val
   1               5                   10
<210> 38
   <211> 11
   <212> PRT
   <213> Artificial Sequence
<220>
   <223> Synthetic Peptide
<400> 38
   Ser Ala Ser Gln Asp Ile Ser Asn Tyr Leu Asn
   1               5                   10
<210> 39
   <211> 7
   <212> PRT
   <213> Artificial Sequence
<220>
   <223> Synthetic Peptide
<400> 39
   Phe Thr Ser Ser Leu His Ser
   1               5
<210> 40
   <211> 9
   <212> PRT
   <213> Artificial Sequence
<220>
   <223> Synthetic Peptide
<400> 40
   Gln Gln Tyr Ser Thr Val Pro Trp Thr
   1               5